# EUROPEAN PATENT APPLICATION

(11) **EP 4 656 588 A2**
(43) Date of publication of application: **03.12.2025**
(21) Application number: 25195393.1
(22) Date of filing: 09.12.2021
(51) Int. Cl.: B82Y 5/00

(54) **ENHANCED NANOPARTICLE DELIVERY SYSTEMS**

(30) Priority: 10.12.2020 US 202063123519 P
(62) Divisional of application: 21904402.1
(71) Applicant: Children's Hospital Medical Center, Cincinnati, OH 45229-3039 (US)
(72) Inventor: ZIADY, Assem G., Newport, 41071 (US); SIEFERT, Mathew, Cincinnati, 45231 (US)
(74) Representative: Forresters IP LLP

(57) **Abstract**

Disclosed are methods for the enhancement of nucleic acid delivery systems. The methods may employ treatment with a compound and/or an RNAi molecule in combination with a nucleic acid to improve nucleic acid uptake into a cell. In particular, the disclosed methods may be useful for improved gene therapy techniques.

## Description

### CROSS REFERENCE TO RELATED APPLICATION

This application claims the benefit of and priority to U.S. Provisional Application No. 63/123,519, entitled "Enhanced Nanoparticle Delivery Systems," filed December 10, 2020, the contents of which are incorporated by reference in its entirety for all purposes.

### STATEMENT REGARDING FEDERALLY-SPONSORED RESEARCH

This invention was made with government support under EB023800 awarded by the National Institutes of Health. The government has certain rights in this invention.

### BACKGROUND

Transfer of nucleic acids, including double and single stranded DNA as well as RNA, into eukaryotic cells is the most essential step of any gene transfer, repair, or editing technology. Transfer of nucleic acids may be accomplished using many types of delivery vehicles, including cationic lipids, viral vectors and nucleic acid nanoparticles condensed with cationic polymers such as poly lysine or polyethyleneimine. However, significant costs involved in the preparation of these materials present a significant limitation in their usage as both research tools and translational applications such as gene therapy. Further, efficacy of nucleic acid transfer with or without modification of the vector remains an area in need of improvement. The instant disclosure seeks to address one or more of the aforementioned needs in the art.

### BRIEF SUMMARY

Disclosed are methods for the enhancement of nucleic acid delivery systems. The methods may employ treatment with a compound and/or a nucleic acid molecule such as, for example, one or more molecules selected from RNAi, miRNA, shRNA, tRNA, siRNA, single and double stranded DNA in combination with, for example, prior to or concurrent with, administration of a nucleic acid to improve nucleic acid uptake into a cell. In particular, the disclosed methods may be useful for improved gene therapy techniques in which a disclosed RNAi and/or a disclosed compound may be administered prior to or concurrently with the gene therapy delivery vehicle containing a nucleic acid.

### BRIEF DESCRIPTION OF THE DRAWINGS

This application file contains at least one drawing executed in color. Copies of this patent or patent application publication with color drawing(s) will be provided by the Office upon request and payment of the necessary fee.

Those of skill in the art will understand that the drawings, described below, are for illustrative purposes only. The drawings are not intended to limit the scope of the present teachings in any way.

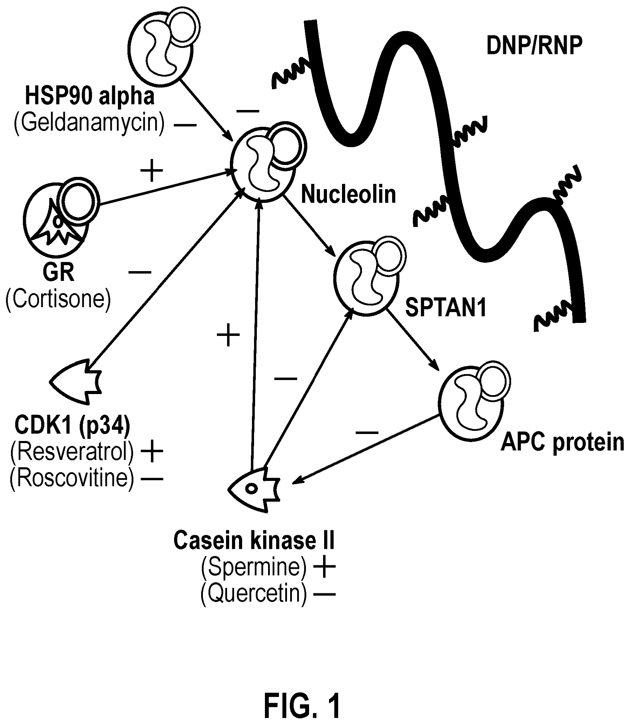

FIG 1 is a schematic of partial NNP (DNP and RNP) interactome including nucleoin, APC, and SPTAN1, which were identified by MS analysis of 2 gel bands from DNP and RNP pull downs not present in bead alone control. Lighter color circles connote interactions that enhance NNP-mediated gene transfer, while darker circles connote interactions that inhibit. (+) or (-) along arrows connote impact on interactions with DNP. (+) or (-) by pharmacological agents reflect impact on the activation of GR, CDK1, or CKII. For example, while cortisone would increase nucleolin at the membrane via GR (10), spermine would increase it through stimulation of CKII mediated phosphorylation of nucleolin. Pull downs initially conducted in primary hepatocytes and repeated three times in wd-AECs for 2 non-CF and 3 CF subjects. This DNP interactome was observed in all the hepatocyte and CF and non-CF wd-AEC studies.

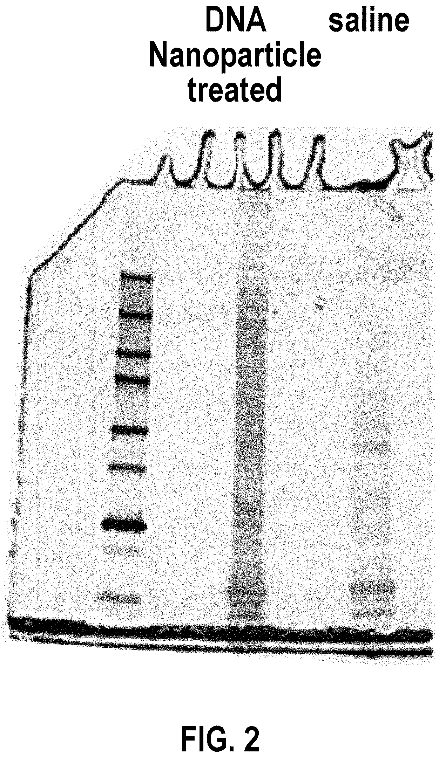

FIG 2 depicts immunoprecipitation of protein interactors of DNA nanoparticles in HeLa cells.

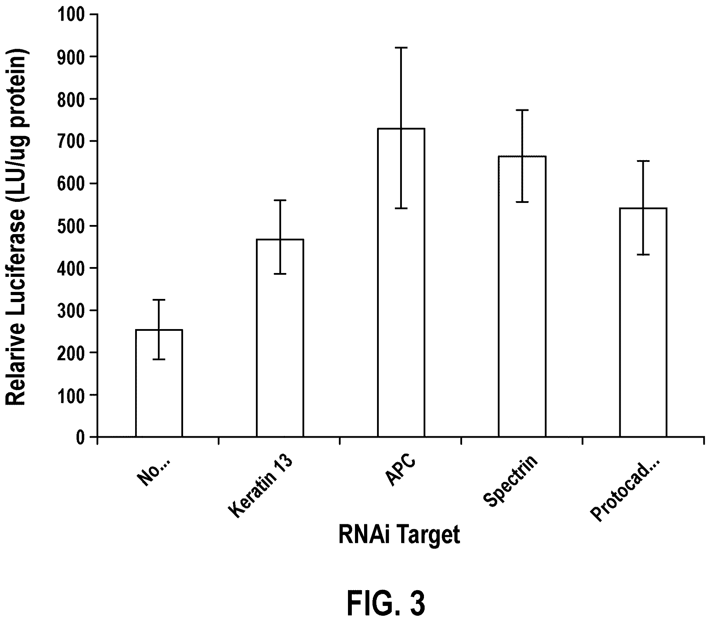

FIG 3 depicts enhanced DNA nanoparticle transfection through siRNA expression.

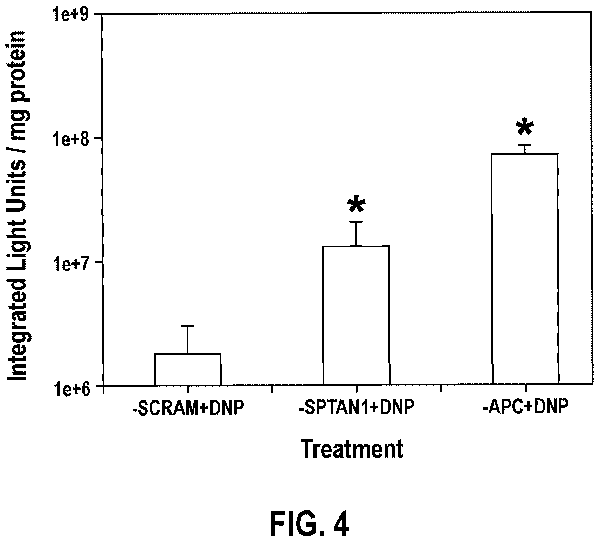

FIG 4 depicts transfection of human primary airway epithelia either following prior treatment with scrambled shRNA, shRNA specific for APC, or shRNA specific for SPTAN1 for 48 hours. Luciferase expression was measured two days post transfection. * connotes different from saline pretreatment (triplicates in three experiments p<0.01).

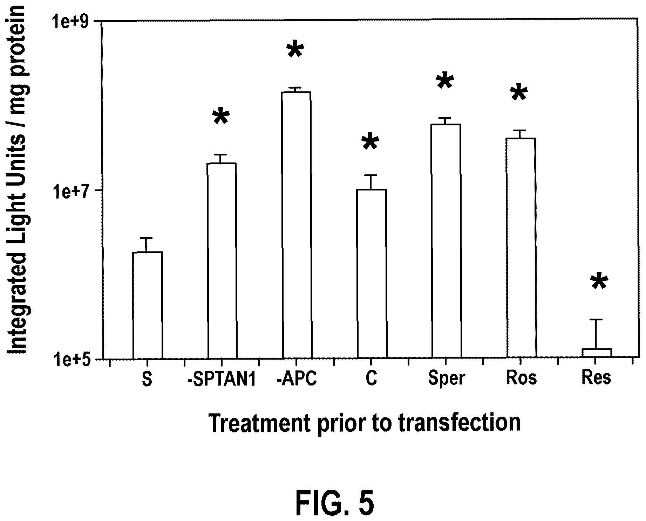

FIG 5 depicts primary cell cultures of airway epithelia transfected with DNPs containing a plasmid coding for luciferase driven by the ubiquitin B promoter (5.4 kb). shRNA lentivirus infection was 48 hours prior to transfection while spermine (CK11 inducer) roscovitine (CDK1 inhibitor), resveratrol (CDK1 agonist), or cortisone (GR agonist) were added four hours prior to transfection. Treatments were saline (S), APC shRNA (-APC), SPTAN1 shRNA (-SPTAN1), hydrocortisone Ⓒ, spermine (Sper), roscovitine (Ros), or resveratrol (RES). Luciferase expression was measured two days post transfection. * connotes different from saline (p<0.01).

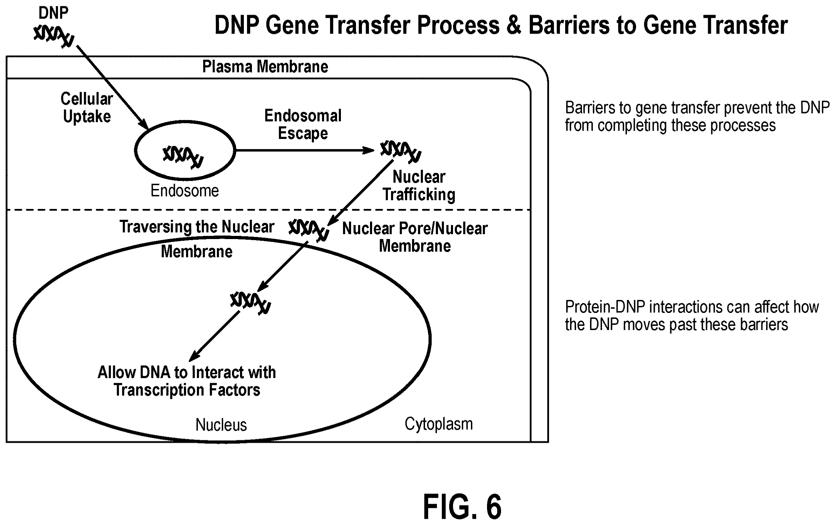

FIG 6 is a schematic showing DNP Gene Transfer Process & Barriers to Gene Transfer. Barriers to gene transfer prevent the DNP from completing these processes. Protein-DNP interactions can affect how the DNP moves past these barriers.

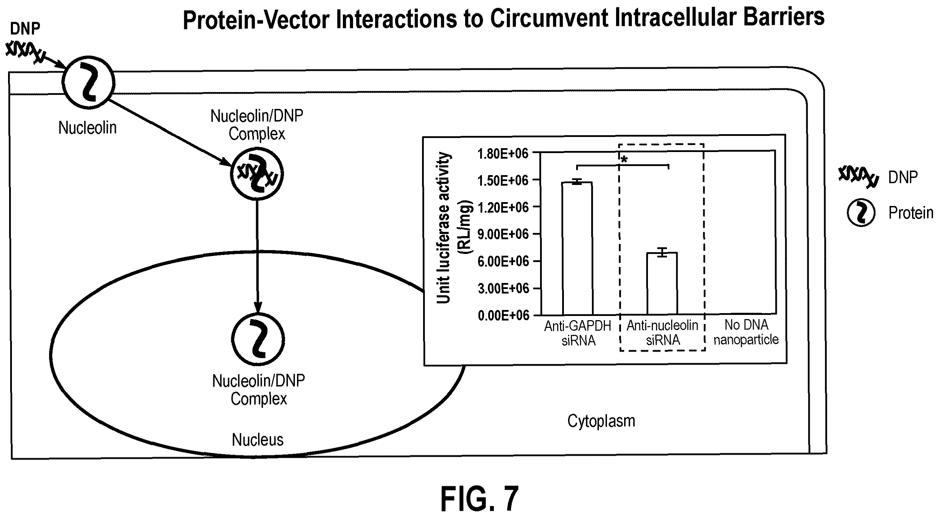

FIG 7 is a schematic showing Protein-Vector Interactions to Circumvent Intracellular Barriers.

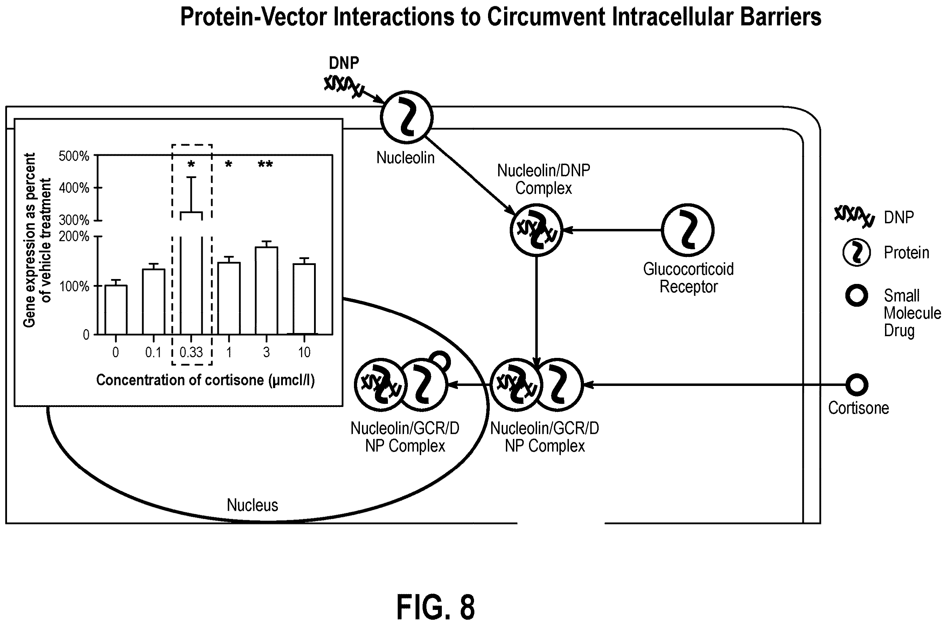

FIG 8 is a schematic showing Protein-Vector Interactions to Circumvent Intracellular Barriers. Concentration of cortisone vs gene expression as a percent of vehicle treatment.

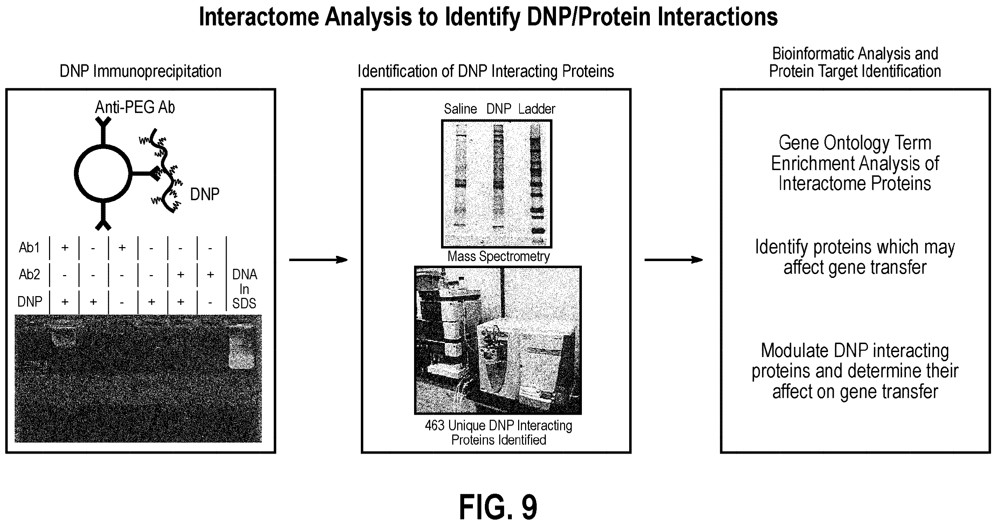

FIG 9. Interactome Analysis to Identify DNP/Protein Interactions

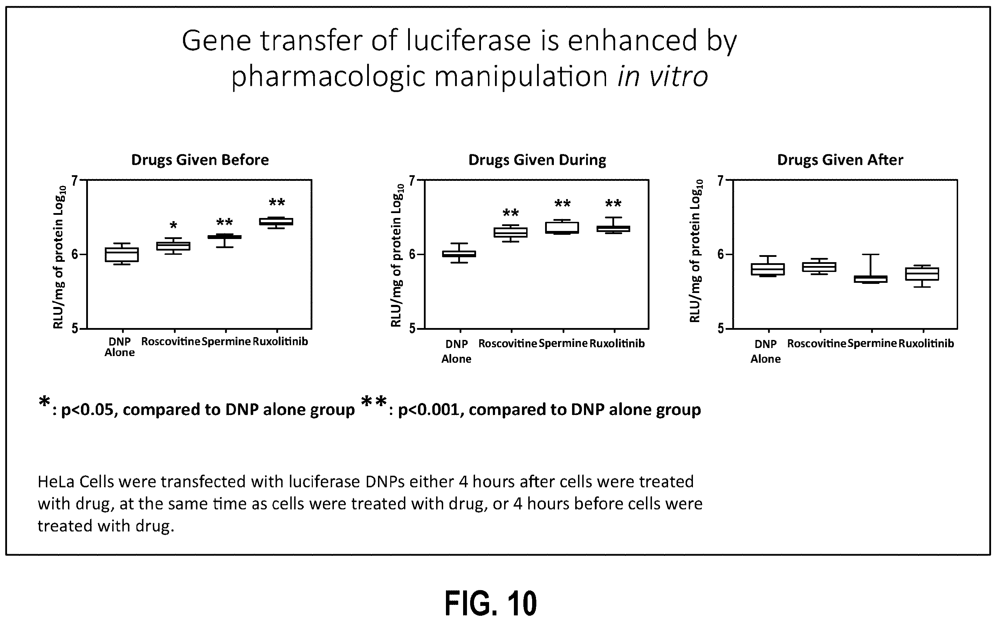

FIG 10 depicts graphs showing that gene transfer of luciferase is enhanced by pharmacologic manipulation *in vitro.* HeLa cells were transfected with luciferase DNPs either four hours after cells were treated with drug, at the same time as cells were treated with drug, or four hours before cells were treated with drug; DNP alone, RX001 (roscovitine), RX011 (spermine), and RX008 (ruxolitinib).

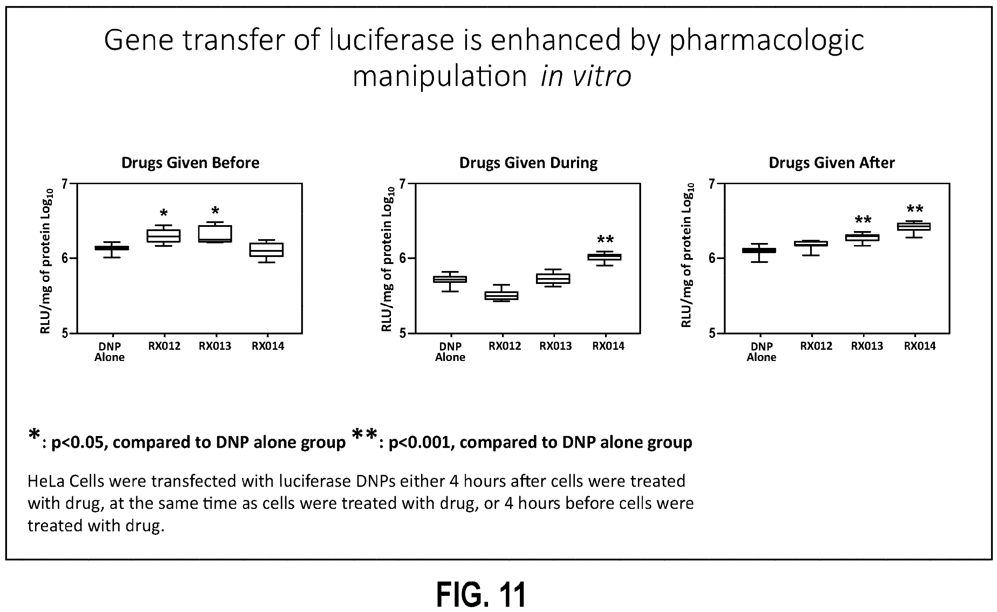

FIG 11 depicts graphs showing that gene transfer of luciferase is enhanced by pharmacologic manipulation in vitro. HeLa cells were transfected with luciferase DNPs either four hours after cells were treated with drug, at the same time as cells were treated with drug, or four hous before cells were treated with drug, DNP alone, RX012 (doxorubicin), RX013 (acetohexamide), and RX014 (sildenafil citrate).

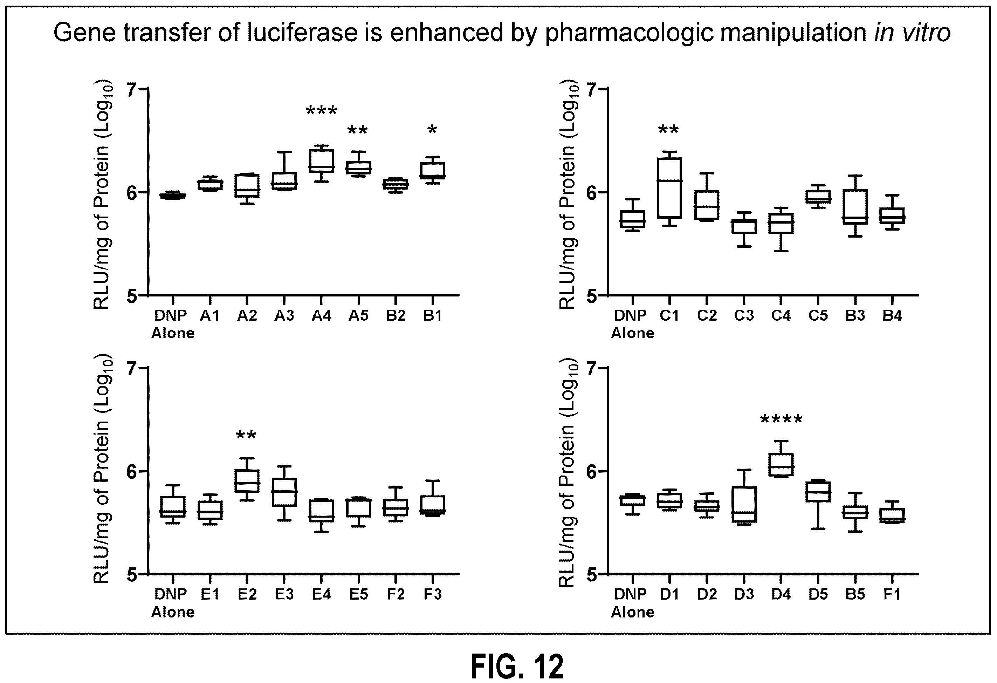

FIG 12. Gene transfer of luciferase is enhanced by pharmacologic manipulation in vitro. HeLa cell were treated for four hours prior to luciferase DNP administration. Drugs were obtained from a blinded plate. Labels on graphs indicate drugs position on blinded plate. *: p<0.05, **: p<0.01,***: p<0.001, ***: p<0.0001, compared to DNP Alone group .

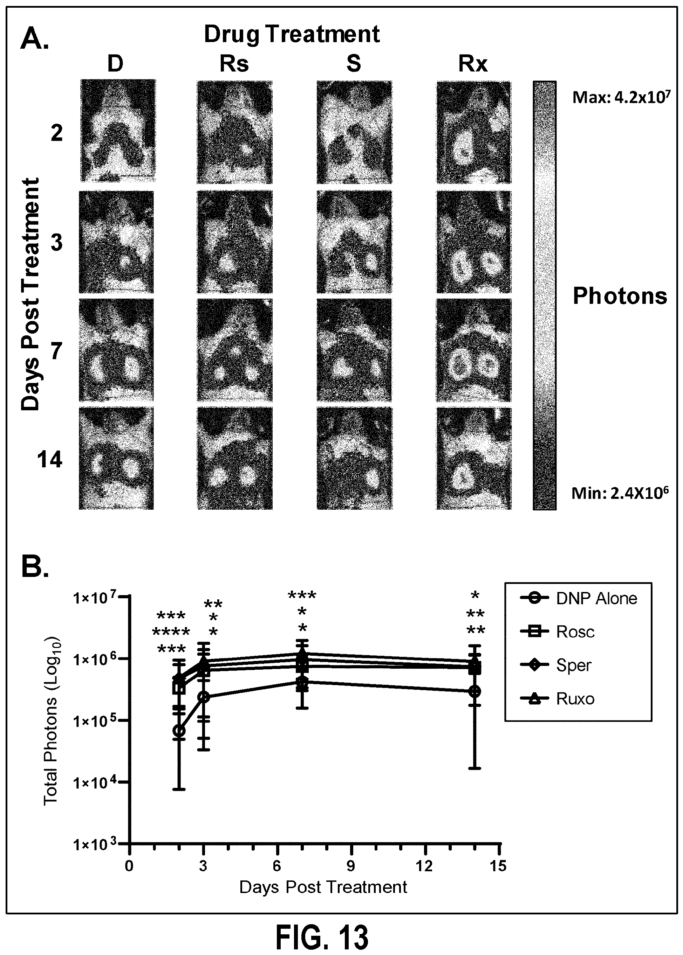

FIG 13 shows that enhancement of in vivo DNP transfection by pharmaceuticals is maintained over time. Roscovitine (10 mg/kg, CDK1 inhibitor), spermine (20 mg/kg, CKII activator), and ruxolitinib (1 mg/kg, JAK inhibitor) were dosed in mice 2 hours prior to intratracheal administration of 100 µg luciferase DNP. A. BLI images taken at 2, 3, 7, and 14 days after DNP dosage of a single mouse from groups of mice given DNP only (D), roscovitine (Rs), spermine (S), and ruxolitinib (Rx). Total photon measurements were taken from the chest of each mouse, ROI outlined in red, and background luminescence was subtracted from mice saline control mice (not shown). B. Total photons collected over a 10 min BLI exposure from the ROI at each day. (n>10 for each group, mean and SD shown, p<0.05, p<0.01, p<0.001 compared to the DNP alone group) Note: At day 14, spermine was significantly different (P<0.5) from the DNP alone group as well as roscovitine (p<0.01). Mean graphed with SD, significance for each time point is in the order of ruxo, sper, rose (top to bottom). *: p<0.05, **: p<0.01,***: p<0.001, ***: p<0.0001, compared to the DNP Alone group at the same day post treatment.

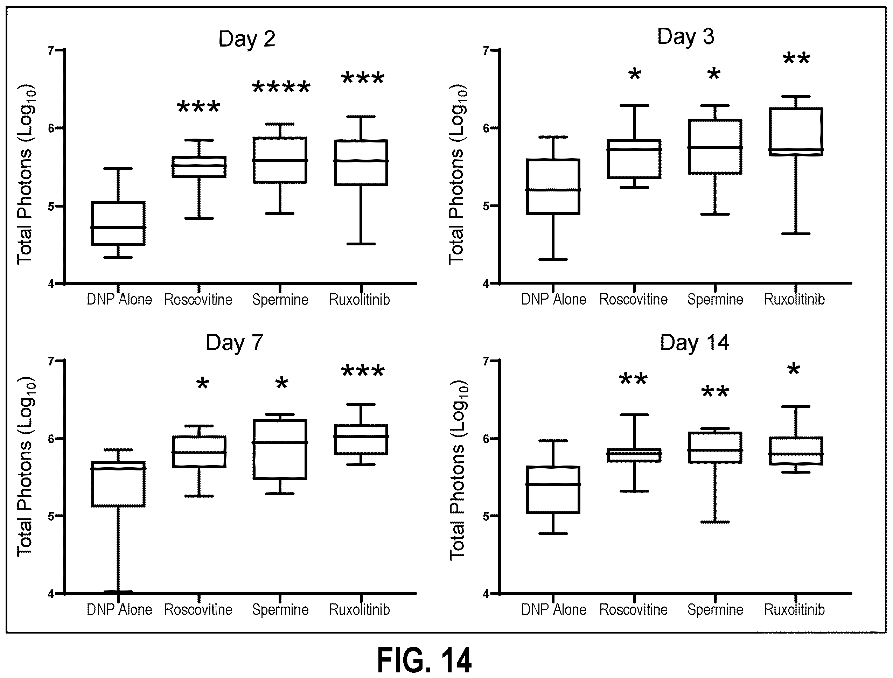

FIG 14 shows that pharmacological manipulation of interactome proteins enhances luciferase gene transfer in vivo. Bioluminescent Image (BLI) analysis, RX001, RX011, RX008, day 2, day 3, day 7, and day 14. *: p<0.05, **: p<0.01, ***: p<0.001, ***: p<0.0001, compared to the DNP Alone group at the same day post treatment.

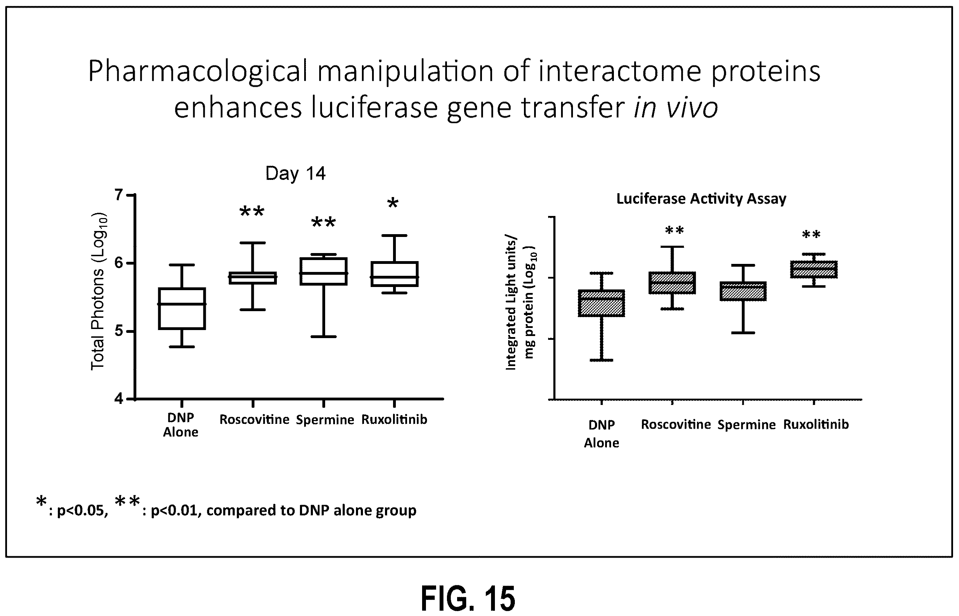

FIG 15 shows that pharmacological manipulation of interactome proteins enhances luciferase gene transfer in vivo BLI image analysis and luciferase activity assay for DNP alone, RX001 (roscovitine), RX011 (spermine), and RX008 (ruxolitinib).

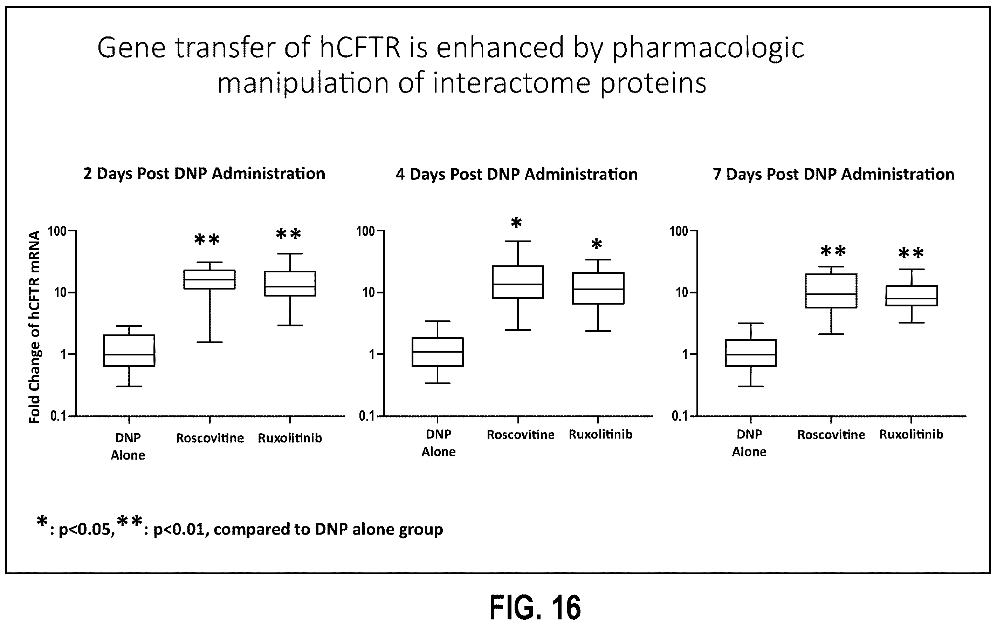

FIG 16. Gene transfer of hCFTR is enhanced by pharmacologic manipulation of interactome proteins. DMP alone, RX001 (roscovitine), and RX008 (ruxolitinib), 2 days post-DNP administration, 4 days post-DNP administration and 7 days post-DNP administration.

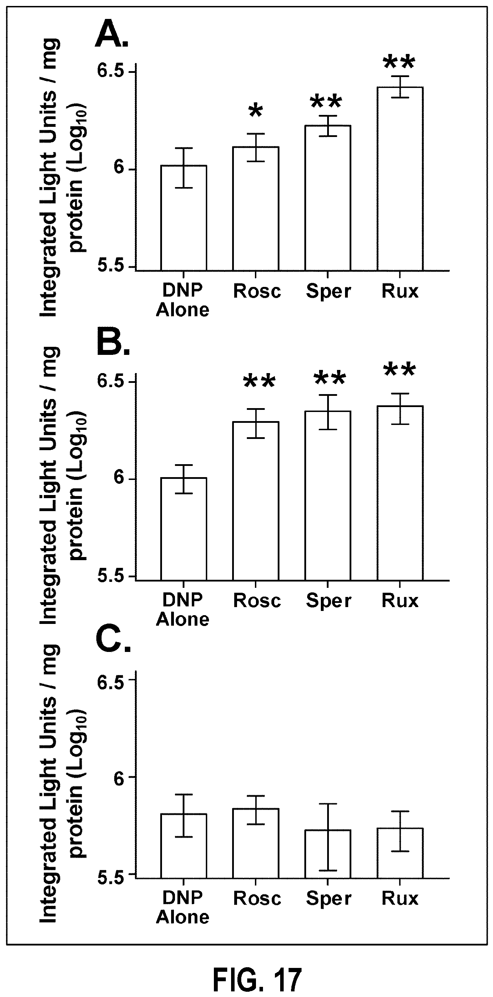

FIG 17 depicts pharmacological manipulations before, after, and during DNP transfection. Hela cells were given roscovitine (1 µM, CDK1 inhibitor), spermine (1 µM, CKII activator), or ruxolitinib (0.1 µM, JAK inhibitor) at various times during transfection of luciferase DNPs . A. Hela cells were dosed with drugs for 4 hours, washed, and then given luciferase DNPs for 24 hours. B. Hela cells were simultaneously given drug and luciferase DNPs for 24 hr. C. Hela cells were given luciferase DNPs for 4 hours, washed, and then given drug for 24 hr. All cells were then lysed and analyzed for luciferase activity with a light-based assay. (n=8 for each group, signifies p<0.05 and signifies p<0.001).

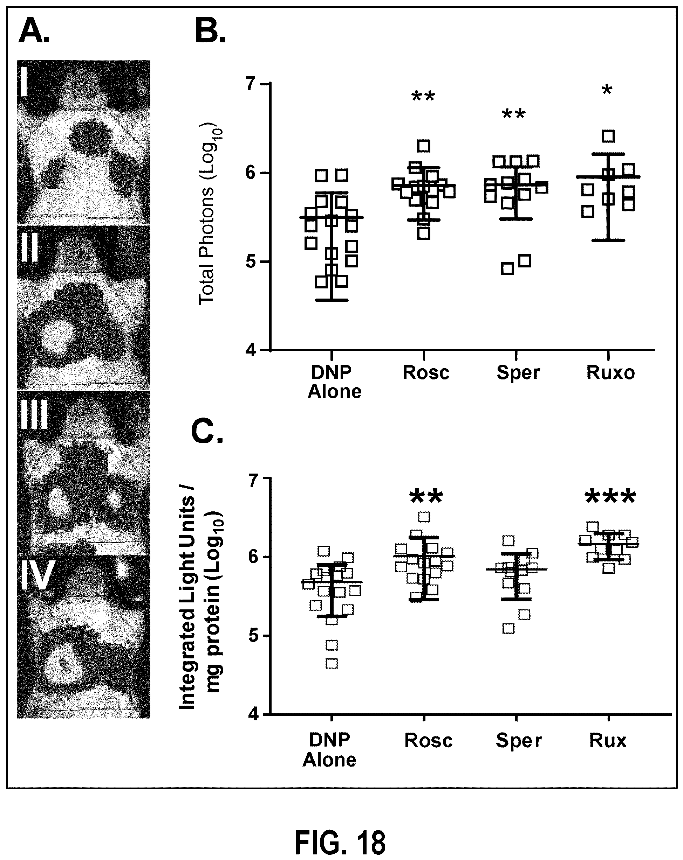

FIG 18 shows pharmaceutical enhancement of DNP gene delivery efficacy in vivo. Roscovitine (10 mg/kg, CDK1 inhibitor), spermine (20 mg/kg, CKII activator), and ruxolitinib (1 mg/kg, JAK inhibitor) were dosed in mice 2 hours prior to intratracheal administration of 100 µg luciferase DNP. All data was collected 14 days post DNP administration. A. representative BLI images of mice given I) DNP only, II) Roscovitine, III) Spermine, and IV) ruxolitinib. The ROI, outlined in red, was consistently drawn on each mouse and used to quantify total photons in each mouse. B. Total photons collected over a 10 min BLI exposure from the ROI. Mice that received saline instead of DNPs (not shown) were used to subtract background from the experimental mice. C. Lungs from mice used in B were harvested immediately after BLI imaging and assayed for luciferase activity. (n>10 for each group, p<0.05, p<0.01, p<0.001)

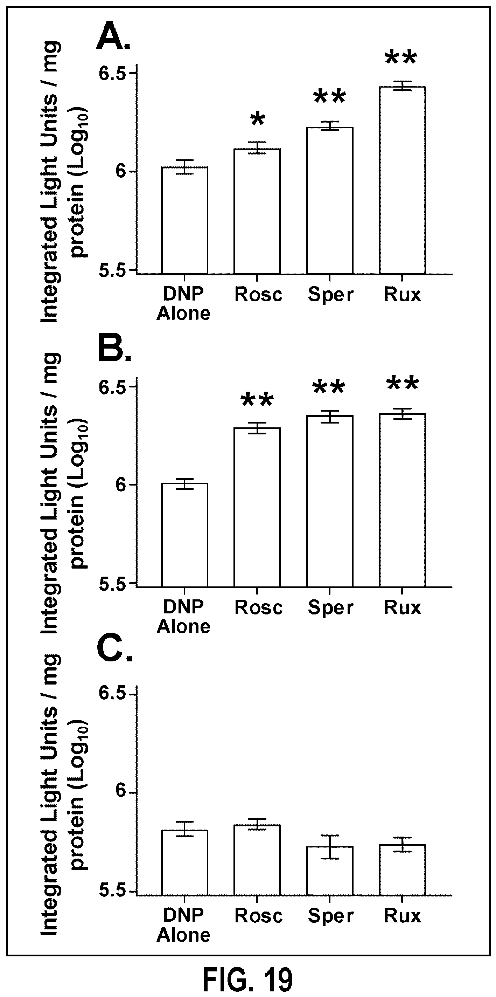

FIG 19 shows pharmacological manipulations before, after, and during DNP transfection. Hela cells were given roscovitine (1 µM, CDK1 inhibitor), spermine (1 µM, CKII activator), or ruxolitinib (0.1 µM, JAK inhibitor) at various times during transfection of luciferase DNPs. A. Hela cells were dosed with drugs for 4 hours, washed, and then given luciferase DNPs for 24 hours. B. Hela cells were simultaneously given drug and luciferase DNPs for 24 hr. C. Hela cells were given luciferase DNPs for 4 hours, washed, and then given drug for 24 hr. All cells were then lysed and analyzed for luciferase activity with a light-based assay. (n=8 for each group, mean and SEM shown, signifies p<0.05 and signifies p<0.001)

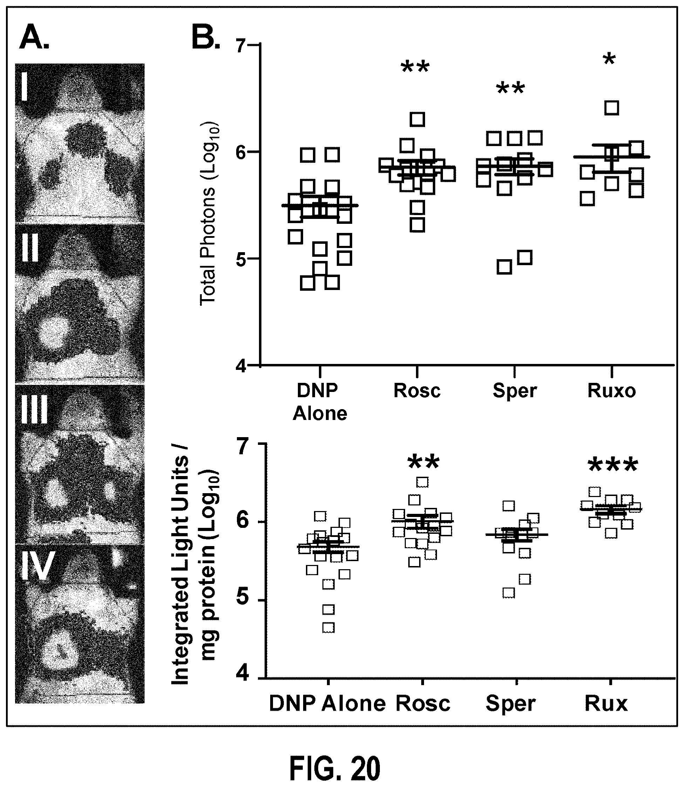

FIG 20 depicts pharmaceutical enhancement of DNP gene delivery efficacy in Vivo. Roscovitine (10 mg/kg, CDK1 inhibitor), spermine (20 mg/kg, CKII activator), and ruxolitinib (1 mg/kg, JAK inhibitor) were dosed in mice 2 hours prior to intratracheal administration of 100 µg luciferase DNP. All data was collected 14 days post DNP administration. A. representative BLI images of mice given I) DNP only, II) Roscovitine, III) Spermine, and IV) ruxolitinib. The ROI, outlined in red, was consistently drawn on each mouse and used to quantify total photons in each mouse. B. Total photons collected over a 10 min BLI exposure from the ROI. Mice that received saline instead of DNPs (not shown) were used to subtract background from the experimental mice. C. Lungs from mice used in B were harvested immediately after BLI imaging and assayed for luciferase activity. (n>10 for each group, mean and SEM shown, p<0.05, p<0.01, p<0.001)

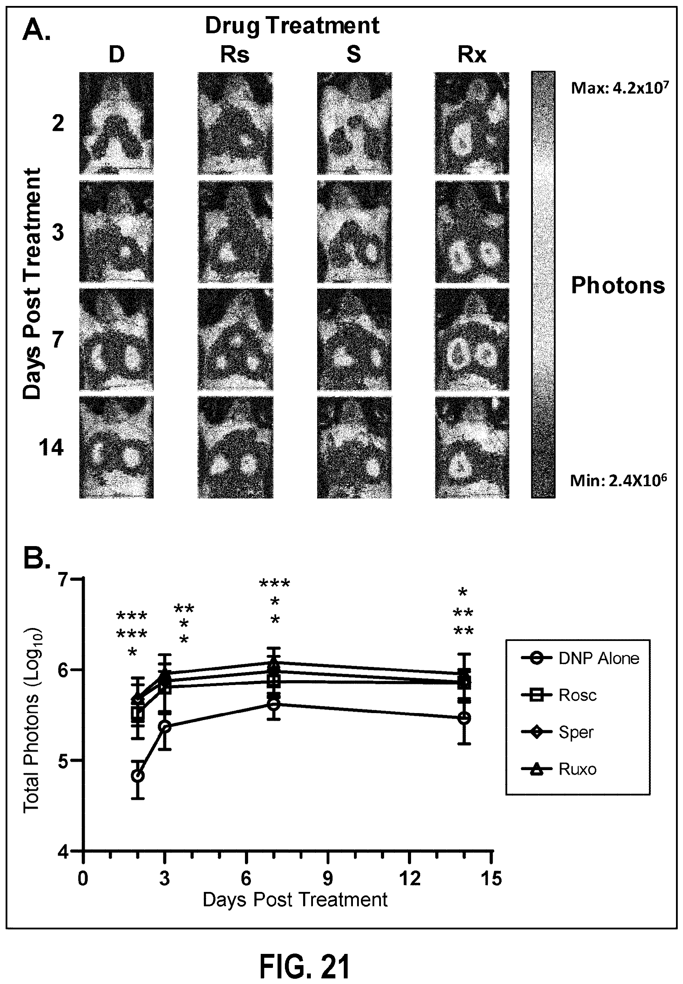

FIG 21 shows that enhancement of in vivo DNP transfection by pharmaceuticals is maintained over time. Roscovitine (10 mg/kg, CDK1 inhibitor), spermine (20 mg/kg, CKII activator), and ruxolitinib (1 mg/kg, JAK inhibitor) were dosed in mice 2 hours prior to intratracheal administration of 100 µg luciferase DNP. A. BLI images taken at 2, 3, 7, and 14 days after DNP dosage of a single mouse from groups of mice given DNP only (D), roscovitine (Rs), spermine (S), and ruxolitinib (Rx). Total photon measurements were taken from the chest of each mouse, ROI outlined in red, and background luminescence was subtracted from mice saline control mice (not shown). B. Total photons collected over a 10 min BLI exposure from the ROI at each day. (n>10 for each group, mean and SEM shown, p<0.05, p<0.01, p<0.001 compared to the DNP alone group) Note: At day 14, spermine was significantly different (P<0.5) from the DNP alone group as well as roscovitine (p<0.01). Mean graphed with SEM, significance for each time point is in the order of ruxo, sper, rose (top to bottom). *: p<0.05, **: p<0.01,***: p<0.001, ***: p<0.0001, compared to the DNP Alone group at the same day post treatment.

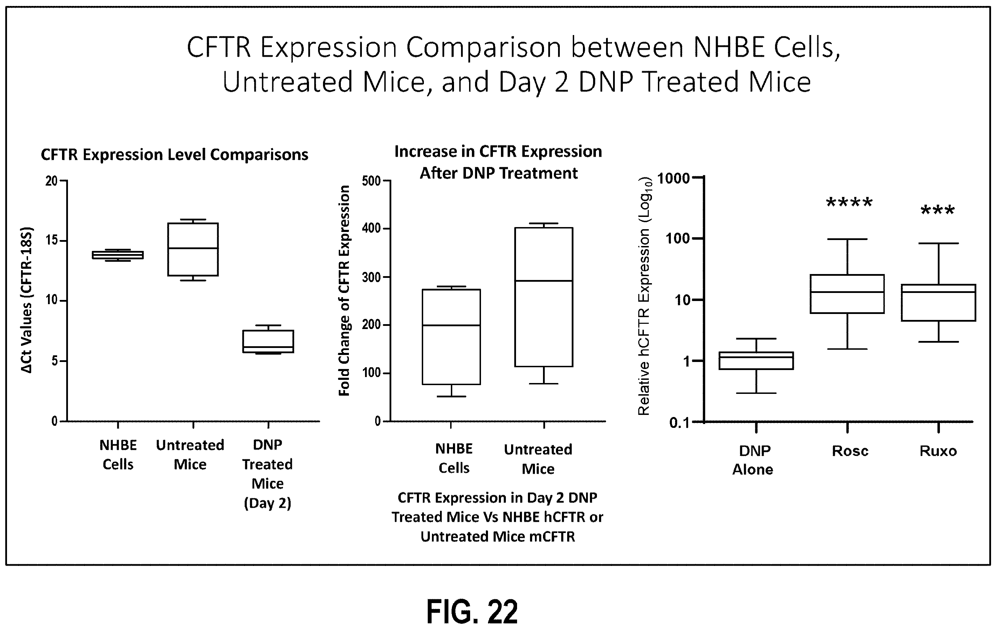

FIG 22. CFTR Expression comparison between NHBE cells (normal human bronchial/tracheal epithelial cells), Untreated Mice and Day 2 DNP Treated Mice, and CFTR Expression comparison in Day 4 mice treated with a vehicle (DNP Alone) or drug. Day 4 data represents vector subtracted values for the PCR of CFTR.

### DETAILED DESCRIPTION

### DEFINITIONS

Unless otherwise noted, terms are to be understood according to conventional usage by those of ordinary skill in the relevant art. In case of conflict, the present document, including definitions, will control. Preferred methods and materials are described below, although methods and materials similar or equivalent to those described herein may be used in practice or testing of the present invention. All publications, patent applications, patents and other references mentioned herein are incorporated by reference in their entirety. The materials, methods, and examples disclosed herein are illustrative only and not intended to be limiting.

As used herein and in the appended claims, the singular forms "a," "and," and "the" include plural referents unless the context clearly dictates otherwise. Thus, for example, reference to "a method" includes a plurality of such methods and reference to "a dose" includes reference to one or more doses and equivalents thereof known to those skilled in the art, and so forth.

The term "about" or "approximately" means within an acceptable error range for the particular value as determined by one of ordinary skill in the art, which will depend in part on how the value is measured or determined, e.g., the limitations of the measurement system. For example, "about" may mean within 1 or more than 1 standard deviation, per the practice in the art. Alternatively, "about" may mean a range of up to 20%, or up to 10%, or up to 5%, or up to 1% of a given value. Alternatively, particularly with respect to biological systems or processes, the term may mean within an order of magnitude, preferably within 5-fold, and more preferably within 2-fold, of a value. Where particular values are described in the application and claims, unless otherwise stated the term "about" meaning within an acceptable error range for the particular value should be assumed.

As used herein, the term "effective amount" means the amount of one or more active components that is sufficient to show a desired effect. This includes both therapeutic and prophylactic effects. When applied to an individual active ingredient, administered alone, the term refers to that ingredient alone. When applied to a combination, the term refers to combined amounts of the active ingredients that result in the therapeutic effect, whether administered in combination, serially or simultaneously.

The terms "individual," "host," "subject," and "patient" are used interchangeably to refer to an animal that is the object of treatment, observation and/or experiment. Generally, the term refers to a human patient, but the methods and compositions may be equally applicable to non-human subjects such as other mammals. In some embodiments, the terms refer to humans. In further embodiments, the terms may refer to children.

As used herein, a "pharmaceutically acceptable form thereof" includes any pharmaceutically acceptable salts, prodrugs, tautomers, isomers, and/or isotopically labeled derivatives of a compound provided herein, as defined below and herein.

The term "pharmaceutically acceptable salt," as used herein, refers to a formulation of a compound that does not cause significant irritation to an organism to which it is administered and does not abrogate the biological activity and properties of the compounds described herein. As used herein, the disclosed compounds also include pharmaceutically acceptable salts thereof.

As used herein, the term "prodrug" refers to a derivative of a parent compound that requires transformation within the body in order to release the parent compound. A prodrug can be inactive when administered to a subject, but is converted in vivo to an active compound, for example, by hydrolysis (e.g., hydrolysis in blood). In certain cases, a prodrug has improved physical and/or delivery properties over the parent compound. Prodrugs are typically designed to enhance pharmaceutically and/or pharmacokinetically based properties associated with the parent compound. The advantage of a prodrug can lie in its physical properties, such as enhanced water solubility for parenteral administration at physiological pH compared to the parent compound, or it enhances absorption from the digestive tract, or it can enhance drug stability for long-term storage. (see, e.g., Bundgard, H., Design of Prodrugs (1985), pp. 7-9, 21-24 (Elsevier, Amsterdam). A discussion of prodrugs is provided in Higuchi, T., et al., "Pro-drugs as Novel Delivery Systems," A.C.S. Symposium Series, Vol. 14, and in Bioreversible Carriers in Drug Design, ed. Edward B. Roche, American Pharmaceutical Association and Pergamon Press, 1987, both of which are incorporated in full by reference herein.

The term "prodrug" is also meant to include any covalently bonded carriers, which release the active compound in vivo when such prodrug is administered to a subject. Prodrugs of an active compound, as described herein, can be prepared by modifying functional groups present in the active compound in such a way that the modifications are cleaved, either in routine manipulation or in vivo, to the parent active compound. Prodrugs include compounds wherein a hydroxy, amino or mercapto group is bonded to any group that, when the prodrug of the active compound is administered to a subject, cleaves to form a free hydroxy, free amino or free mercapto group, respectively. Examples of prodrugs include, but are not limited to, acetate, formate and benzoate derivatives of an alcohol or acetamide, formamide and benzamide derivatives of an amine functional group in the active compound and the like. Other examples of prodrugs include compounds that comprise -NO, -NO₂, - ONO, or -ONO₂ moieties. Prodrugs can typically be prepared using well-known methods, such as those described in Burger's Medicinal Chemistry and Drug Discovery, 172-178, 949-982 (Manfred E. Wolff ed., 5th ed., 1995), and Design of Prodrugs (H. Bundgaard ed., Elselvier, N.Y., 1985).

For example, if a disclosed compound or a pharmaceutically acceptable form of the compound contains a carboxylic acid functional group, a prodrug can comprise a pharmaceutically acceptable ester formed by the replacement of the hydrogen atom of the acid group with a group such as (C₁-C₈)alkyl, (C₂-C₁₂)alkanoyloxymethyl, 1-(alkanoyloxy)ethyl having from 4 to 9 carbon atoms, 1-methyl-1-(alkanoyloxy)-ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)amino)ethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as (3-dimethylaminoethyl), carbamoyl-(C₁-C₂)alkyl, N,N-di(C₁-C₂)alkylcarbamoyl-(C₁-C₂)alkyl and piperidino-, pyrrolidino- or morpholino(C₂-C₃)alkyl.

Similarly, if a disclosed compound or a pharmaceutically acceptable form of the compound contains an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1-((C₁-C₆)alkanoyloxy)ethyl (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, P(O)(OH)₂, -P(O)(O(C₁-C₆)alkyl)₂ or glycosyl (the radical resulting from the removal of a hydroxyl group of the hemiacetal form of a carbohydrate).

If a disclosed compound or a pharmaceutically acceptable form of the compound incorporates an amine functional group, a prodrug can be formed by the replacement of a hydrogen atom in the amine group with a group such as R-carbonyl, RO-carbonyl, NRR'-carbonyl where R and R' are each independently (C₁-C₁₀)alkyl, (C₃-C₇)cycloalkyl, benzyl, a natural α-aminoacyl or natural α-aminoacyl-natural α-aminoacyl, -C(OH)C(O)OY¹ wherein Y¹ is H, (C₁-C₆)alkyl or benzyl, -C(OY²)Y³ wherein Y² is (C₁-C₄) alkyl and Y³ is (C₁-C₆)alkyl, carboxy(C₁-C₆)alkyl, amino(C₁-C₄)alkyl or mono-N- or di-N,N-(C₁-C₆)alkylaminoalkyl, C(Y⁴)Y⁵ wherein Y⁴ is H or methyl and Y⁵ is mono-N- or di-N,N-(C₁-C₆)alkylamino, morpholino, piperidin-1-yl or pyrrolidin-1-yl.

The active agent may form salts, which are also within the scope of the preferred embodiments. Reference to a compound of the active agent herein is understood to include reference to salts thereof, unless otherwise indicated. The term "salt(s)", as employed herein, denotes acidic and/or basic salts formed with inorganic and/or organic acids and bases. In addition, when an active agent contains both a basic moiety, such as, but not limited to an amine or a pyridine or imidazole ring, and an acidic moiety, such as, but not limited to a carboxylic acid, zwitterions ("inner salts") may be formed and are included within the term "salt(s)" as used herein. Pharmaceutically acceptable (e.g., non-toxic, physiologically acceptable) salts are preferred, although other salts are also useful, e.g., in isolation or purification steps, which may be employed during preparation. Salts of the compounds of the active agent may be formed, for example, by reacting a compound of the active agent with an amount of acid or base, such as an equivalent amount, in a medium such as one in which the salt precipitates or in an aqueous medium followed by lyophilization. When the compounds are in the forms of salts, they may comprise pharmaceutically acceptable salts. Such salts may include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Examples of metal salts include lithium, sodium, potassium, magnesium salts and the like. Examples of ammonium and alkylated ammonium salts include ammonium, methylammonium, dimethylammonium, trimethylammonium, ethylammonium, hydroxyethylammonium, diethylammonium, butylammonium, tetramethylammonium salts and the like. Examples of organic bases include lysine, arginine, guanidine, diethanolamine, choline and the like.

"Sequence identity" as used herein indicates a nucleic acid sequence that has the same nucleic acid sequence as a reference sequence, or has a specified percentage of nucleotides that are the same at the corresponding location within a reference sequence when the two sequences are optimally aligned. For example, a nucleic acid sequence may have at least 50%, 60%, 70%, 75%, 80%, 85%, 90%, 95%, 96%, 97%, 98%, 99%, or 100% identity to the reference nucleic acid sequence. The length of comparison sequences will generally be at least 5 contiguous nucleotides, preferably at least 10, 11, 12, 13, 14, 15, 16, 17, 18, 19, 20, 21, 22, 23, 24, or 25 contiguous nucleotides, and most preferably the full length nucleotide sequence. Sequence identity may be measured using sequence analysis software on the default setting (e.g., Sequence Analysis Software Package of the Genetics Computer Group, University of Wisconsin Biotechnology Center, 1710 University Avenue, Madison, Wis. 53705). Such software may match similar sequences by assigning degrees of homology to various substitutions, deletions, and other modifications.

The term "NNP" refers to a Nucleic acid Nano Particle. A non-limiting example includes a complex of DNA or RNA with polymers of lysines (for example, 15-45 lysines long).

The term "DNP" refers to a DNA Nanoparticle

The term "RNP" refers to a RNA Nanoparticle

The term "Interactome" refers to the whole set of molecular interactions in a particular cell. The term specifically refers to physical interactions among molecules (such as those among proteins, also known as protein-protein interactions) but can also describe sets of indirect interactions among genes (genetic interactions).

The term "APC" refers to an adenomatous polyposis coli protein

The term "wd-AECs" refer to well-differentiated airway epithelial cells.

The term "SPTAN1" refers to Alpha II-spectrin, also known as Spectrin alpha chain, a protein that in humans is encoded by the SPTAN1 gene. Alpha II-spectrin is expressed in a variety of tissues and is highly expressed in cardiac muscle at Z-disc structures, costameres and at the sarcolemma membrane.

The term "GR" refers to a glucocorticoid receptor

The term "CDK1" refers to cyclin dependent kinase 1

The term "CKII" refers to casein kinase II

The term "Spermine" refers to a polyamine involved in cellular metabolism found in all eukaryotic cells.

The term "shRNA" refers to a small hairpin RNA or short hairpin RNA (shRNA) and is an artificial RNA molecule with a tight hairpin turn that can be used to silence target gene expression via RNA interference (RNAi)

Disclosed herein are methods for the enhancement of nucleic acid delivery systems by combination treatment with one or more compounds as disclosed herein and/or one or more RNAi molecules as disclosed herein. For example, the disclosed methods may be used with delivery of a nucleic acid such as a gene, a gene fragment, a fragment containing an active portion of a protein encoded by a gene, or the like. Further examples of nucleic acids that may be delivered include nucleic acid components of the CRISPR/CAS9, or short nucleic acids, such as microRNA or DNA or RNA oligonucleotides. The disclosed RNAi molecules and/or compounds may be administered to an individual in need of administration of a nucleic acid prior to administration of a nucleic acid delivery system, or concurrently with the administration of a nucleic acid delivery system.

In one aspect, the method may be a method for transferring a gene into a eukaryotic cell, in which the method may comprise administering a compacted nucleic acid nanoparticle and one or more agents selected from sonolisib (Steroid Lactone), LY294002 (Benzopyran), TG100115 (Pteridine), Trifluoperazine (Benzothiazine, Phenothiazine), CEP5214 (Indole Derivative, Pyrrolocarbazole), Afuresertib (Substituted Benzene, Phenethylamine, Amphetamine), Cation Vemurafenib (Aryl-Phenylketone), Suramin (Benzene Derivative, Benzanilide), Uprosertib (Benzene Derivative, Phenethylamine, Amphetamine), Flutamide, (Benzene Derivative, Trifluoromethylbenzene), Enzastaurin (Indole Derivative, N-alkylindole), Fasudil (Isoquinoline derivative), Ruboxistaurin (Macrolactam), Pentamidine (Phenol Ether), Uprosertib (Benzene Derivative, Phenethylamine, Amphetamine), Lestaurtinib (Indole derivative, Indolocarbazole), Adenine (Imidazolepyrimidine), Pimozide (Diphenylbutylpiperidine), Chlorpromazine (Phenothiazine), Afuresertib (Benzene Derivative, Phenethylamine, Amphetamine), and combinations thereof, to a eukaryotic cell.

In one aspect, the method may comprise administering an inhibitor of a protein that inhibits nanoparticle delivery uptake. In this aspect, the inhibitor may be selected from one or more of RNAi, miRNA, shRNA, tRNA, siRNA, single stranded DNA, double stranded DNA, and combinations thereof. In this aspect, the nucleic acid may inhibit synthesis of one or more proteins that inhibit nucleic acid delivery vehicle uptake. Exemplary proteins may include one or more protein selected from those of Table 1.

In one aspect, the method may comprise administering an active agent that facilitates compacted nucleic acid nanoparticle uptake into a cell. The active agent may inhibit synthesis of one or more proteins that inhibit nucleic acid delivery vehicle uptake.

In one aspect, the RNAi molecule may inhibit expression of a gene encoding a protein selected from Table 1.

In one aspect, the method may comprise administering a second active agent selected from an agent listed in Table 2 or Table 3.

In one aspect, the active agent may be selected from one or more of roscovitine, geldanamycin, acetohexamide, and ruxolitinib, or a combination thereof.

In one aspect, the nucleic acid delivery vehicle may be a nanoparticle comprising one or more of the aforementioned genes.

In one aspect, the compacted nucleic acid nanoparticle may comprise a nucleic acid plasmid and a polymer, wherein the nanoparticle may be compacted in the presence of a counter ion selected from trifluoroacetate (TFA), bromide, bicarbonate, glutamate, hydroxyl ions or combinations thereof.

In one aspect, the nucleic acid may be single or double stranded DNA, or a combination thereof.

In one aspect, the polymer may be a polycation. In one aspect, the polycation may be a lipid. In further aspects, the polycation may be a cysteine (C) containing polymer of lysine (K), such as CK30, a cysteine (C) containing polymer of arginine (R), such as CR30, or combinations thereof. In further aspects, the polycation may be selected from a cysteine (C) containing polymer of lysine (K) and arginine (R), such as C(K5R)5 or C(R5K)5 (e.g. CK15-90), polymers of arginine (e.g. CR15-90), or polymers of lysine mixed with arginine (e.g. C(KR5KR5KR5KR5KR5) or C(K5RK5RK5RK5RK5R)) conjugated to PEG and complexed with nucleic acids. In a further aspect, the polymer may be a lysine polymer, for example a polyethylene glycol (PEG)-substituted lysine polymer or polyethylenemine.

In one aspect, the compacted nucleic acid nanoparticle may have a shape selected from rod shape, ellipsoidal, spheroidal, or toroidal, and may have a diameter of from about 25 to about 400 nm in length as measured by electron microscopy.

The method, in certain aspects, may comprise the steps of
contacting a cell with an RNAi molecule or an active agent. The RNAi molecule or active agent may be in an amount sufficient to inhibit synthesis of one or more proteins that inhibit nucleic acid delivery vehicle uptake; and
contacting the eukaryotic cell with a nucleic acid delivery vehicle.

The cell may be, for example, a eukaryotic cell, derived from a human being.

In one aspect, the disclosed methods may be used to treat an individual in need of such treatment. The individual may be one in which administration a therapeutically effective amount of a protein may be advantageous to reversal, prevention, or amelioration of a disease state. The delivery of a protein may be achieved via administration of a gene, or portion of a gene that encodes an active portion of a protein, that may be subsequently expressed in the individual to provide a functional protein or functional protein fragment in a therapeutically effective amount. In this aspect, the method may comprise the steps of administering an RNAi that inhibits expression of a gene encoding a protein selected from a protein of Table 1 and/or a compound selected from Table 2 or 3, and/or an agent selected from sonolisib (Steroid Lactone), LY294002 (Benzopyran), TG100115 (Pteridine), Trifluoperazine (Benzothiazine, Phenothiazine), CEP5214 (Indole Derivative , Pyrrolocarbazole), Afuresertib (Substituted Benzene, Phenethylamine, Amphetamine), Cation Vemurafenib (Aryl-Phenylketone), Suramin (Benzene Derivative, Benzanilide), Uprosertib (Benzene Derivative, Phenethylamine, Amphetamine), Flutamide, (Benzene Derivative, Trifluoromethylbenzene), Enzastaurin (Indole Derivative, N-alkylindole), Fasudil (Isoquinoline derivative), Ruboxistaurin (Macrolactam), Pentamidine (Phenol Ether), Uprosertib (Benzene Derivative, Phenethylamine, Amphetamine), Lestaurtinib (Indole derivative, Indolocarbazole), Adenine (Imidazolepyrimidine), Pimozide (Diphenylbutylpiperidine), Chlorpromazine (Phenothiazine), Afuresertib (Benzene Derivative, Phenethylamine, Amphetamine), and combinations thereof. The RNAi or agent may be administered concurrently, before, or after administration of a drug delivery vehicle containing the nucleic acid that encodes the gene, or in some instances, the active portion of a gene, of interest.

The amount of compound and/or RNAi necessary to effect the methods of the instant disclosure may be determined by one of ordinary skill in the art. The dose administered to a subject, particularly a human, may be sufficient to effect the desired response in the subject over a reasonable period of time. The dose may be determined by the strength of the particular compound employed and the condition of the subject, as well as the body weight of the subject to be treated. The existence, nature, and extent of any adverse side effects that might accompany the administration of a particular compound also will determine the size of the dose and the particular route of administration employed with a particular patient. For example, the compounds may be therapeutically effective at low doses. Exemplary dosage ranges may be from about 0.001 mM, or less, to about 100 mM, or more, or from about 0.01, 0.05, 0.1, 0.5, 0.6, 0.7, 0.8, or 0.9 mM, to about 1, 2, 3, 4, 5, 6, 7, 8, 9, 10, 20, 30, 40 50, 60, 70, 80, 90 or 100 mM. Accordingly, the compounds may be generally administered in low doses.

In one aspect, the gene is the CF gene, and the individual in need of treatment is an individual having cystic fibrosis.

In one aspect, the RNAi molecule may be one that inhibits expression of a gene encoding a protein selected from a protein of Table 1.

**Table 1. Genes encoding proteins that modulate nucleic acid delivery vehicle uptake. The RNAi molecules of the instant disclosure may inhibit expression of one or more of the genes listed in the table.**

| **Uniprot ID** | **Gene names** | **Protein names** |
|---|---|---|
| P04114 | APOB | Apolipoprotein B-100 (Apo B-100) [Cleaved into: Apolipoprotein B-48 (Apo B-48)] |
| P29536 | LMOD1 | Leiomodin-1 (64 kDa autoantigen 1D) (64 kDa autoantigen 1D3) (64 kDa autoantigen D1) (Leiomodin, muscle form) (Smooth muscle leiomodin) (SM-Lmod) (Thyroid-associated ophthalmopathy autoantigen) |
| P68104 | EEF1A1 EEF1A EF1A LENG7 | Elongation factor 1-alpha 1 (EF-1-alpha-1) (Elongation factor Tu) (EF-Tu) (Eukaryotic elongation factor 1 A-1) (eEF1A-1) (Leukocyte receptor cluster member 7) |
| O19680 | | Pot. HLA-DP-alpha 1 (Aa -31 to +2) (441 is 1st base in codon) (Fragment) |
| P46939 | UTRN DMDL DRP1 | Utrophin (Dystrophin-related protein 1) (DRP-1) |
| P08590 | MYL3 | Myosin light chain 3 (Cardiac myosin light chain 1) (CMLC1) (Myosin light chain 1, slow-twitch muscle B/ventricular isoform) (MLC1SB) (Ventricular myosin alkali light chain) (Ventricular myosin light chain 1) (VLCl) (Ventricular/slow twitch myosin alkali light chain) (MLC-lV/sb) |
| P22695 | UQCRC2 | Cytochrome b-c1 complex subunit 2, mitochondrial (Complex III subunit 2) (Core protein II) (Ubiquinol-cytochrome-c reductase complex core protein 2) |
| Q16763 | UBE2S E2EPF OK/SW-cl.73 | Ubiquitin-conjugating enzyme E2 S (EC 2.3.2.23) (E2 ubiquitin-conjugating enzyme S) (E2-EPF) (Ubiquitin carrier protein S) (Ubiquitin-conjugating enzyme E2-24 kDa) (Ubiquitin-conjugating enzyme E2-EPF5) (Ubiquitin-protein ligase S) |
| P00451 | F8 F8C | Coagulation factor VIII (Antihemophilic factor) (AHF) (Procoagulant component) [Cleaved into: Factor VIIIa heavy chain, 200 kDa isoform; Factor VIIIa heavy chain, 92 kDa isoform; Factor VIII B chain; Factor VIIIa light chain] |
| P52272 | HNRNPM HNRPM NAGR1 | Heterogeneous nuclear ribonucleoprotein M (hnRNP M) |
| P60660 | MYL6 | Myosin light polypeptide 6 (17 kDa myosin light chain) (LC17) (Myosin light chain 3) (MLC-3) (Myosin light chain alkali 3) (Myosin light chain A3) (Smooth muscle and nonmuscle myosin light chain alkali 6) |
| P25054 | APC DP2.5 | Adenomatous polyposis coli protein (Protein APC) (Deleted in polyposis 2.5) |
| P23458 | JAK1 JAK1A JAK1B | Tyrosine-protein kinase JAK1 (EC 2.7.10.2) (Janus kinase 1) (JAK-1) |
| P13533 | MYH6 MYHCA | Myosin-6 (Myosin heavy chain 6) (Myosin heavy chain, cardiac muscle alpha isoform) (MyHC-alpha) |
| P61247 | RPS3A FTE1 MFTL | 40S ribosomal protein S3a (Small ribosomal subunit protein eS1) (v-fos transformation effector protein) (Fte-1) |
| Q08379 | GOLGA2 | Golgin subfamily A member 2 (130 kDa cis-Golgi matrix protein) (GM130) (GM130 autoantigen) (Golgin-95) |
| P41219 | PRPH NEF4 PRPH1 | Peripherin (Neurofilament 4) |
| Q99729 | HNRNPAB ABBP1 HNRPAB | Heterogeneous nuclear ribonucleoprotein A/B (hnRNP A/B) (APOBEC1-binding protein 1) (ABBP-1) |
| P11277 | SPTB SPTB1 | Spectrin beta chain, erythrocytic (Beta-I spectrin) |
| P33981 | TTK MPS1 MPS1L1 | Dual specificity protein kinase TTK (EC 2.7.12.1) (Phosphotyrosine picked threonine-protein kinase) (PYT) |
| P11021 | HSPA5 GRP78 | 78 kDa glucose-regulated protein (GRP-78) (Endoplasmic reticulum lumenal Ca(2+)-binding protein grp78) (Heat shock 70 kDa protein 5) (Immunoglobulin heavy chain-binding protein) (BiP) |
| Q15552 | tb protein | CACCC box-binding protein |
| P62913 | RPL11 | 60S ribosomal protein L11 (CLL-associated antigen KW-12) (Large ribosomal subunit protein uL5) |
| P38919 | EIF4A3 DDX48 KIAA0111 | Eukaryotic initiation factor 4A-III (eIF-4A-III) (eIF4A-III) (EC 3.6.4.13) (ATP-dependent RNA helicase DDX48) (ATP-dependent RNA helicase eIF4A-3) (DEAD box protein 48) (Eukaryotic initiation factor 4A-like NUK-34) (Eukaryotic translation initiation factor 4A isoform 3) (Nuclear matrix protein 265) (NMP 265) (hNMP 265) [Cleaved into: Eukaryotic initiation factor 4A-III, N-terminally processed] |
| Q12905 | ILF2 NF45 PRO3063 | Interleukin enhancer-binding factor 2 (Nuclear factor of activated T-cells 45 kDa) |
| Q14978 | NOLC1 KIAA0035 NS5ATP13 | Nucleolar and coiled-body phosphoprotein 1 (140 kDa nucleolar phosphoprotein) (Nopp140) (Hepatitis C virus NS5A-transactivated protein 13) (HCV NS5A-transactivated protein 13) (Nucleolar 130 kDa protein) (Nucleolar phosphoprotein p130) |
| P20929 | NEB | Nebulin |
| Q16296 | 4R-MAP2 | Microtubule-associated protein (Fragment) |
| P33991 | MCM4 CDC21 | DNA replication licensing factor MCM4 (EC 3.6.4.12) (CDC21 homolog) (P1-CDC21) |
| P49454 | CENPF | Centromere protein F (CENP-F) (AH antigen) (Kinetochore protein CENPF) (Mitosin) |
| Q14008 | CKAP5 KIAA0097 | Cytoskeleton-associated protein 5 (Colonic and hepatic tumor overexpressed gene protein) (Ch-TOG) |
| Q14839 | CHD4 | Chromodomain-helicase-DNA-binding protein 4 (CHD-4) (EC 3.6.4.12) (ATP-dependent helicase CHD4) (Mi-2 autoantigen 218 kDa protein) (Mi2-beta) |
| P55017 | SLC12A3 NCC TSC | Solute carrier family 12 member 3 (Na-Cl cotransporter) (NCC) (Na-Cl symporter) (Thiazide-sensitive sodium-chloride cotransporter) |
| Q92835 | INPP5D SHIP SHIP1 | Phosphatidylinositol 3,4,5-trisphosphate 5-phosphatase 1 (EC 3.1.3.86) (Inositol polyphosphate-5-phosphatase of 145 kDa) (SIP-145) (SH2 domain-containing inositol 5'-phosphatase 1) (SH2 domain-containing inositol phosphatase 1) (SHIP-1) (p150Ship) (hp51CN) |
| Q15269 | PWP2 PWP2H | Periodic tryptophan protein 2 homolog |
| P20585 | MSH3 DUC1 DUG | DNA mismatch repair protein Msh3 (hMSH3) (Divergent upstream protein) (DUP) (Mismatch repair protein 1) (MRP1) |
| Q05086 | UBE3A E6AP EPVE6AP HPVE6A | Ubiquitin-protein ligase E3A (EC 2.3.2.26) (E6AP ubiquitin-protein ligase) (HECT-type ubiquitin transferase E3A) (Human papillomavirus E6-associated protein) (Oncogenic protein-associated protein E6-AP) (Renal carcinoma antigen NY-REN-54) |
| Q92922 | SMARCC1 BAF155 | SWI/SNF complex subunit SMARCC1 (BRG1-associated factor 155) (BAF155) (SWI/SNF complex 155 kDa subunit) (SWI/SNF-related matrix-associated actin-dependent regulator of chromatin subfamily C member 1) |
| P62807 | HIST1H2BC H2BFL; HIST1H2BE H2BFH; HIST1H2BF H2BFG; HIST1H2BG H2BFA; HIST1H2BI H2BFK | Histone H2B type 1-C/E/F/G/I (Histone H2B.1 A) (Histone H2B.a) (H2B/a) (Histone H2B.g) (H2B/g) (Histone H2B.h) (H2B/h) (Histone H2B.k) (H2B/k) (Histone H2B.1) (H2B/1) |
| Q92800 | EZH1 KIAA0388 | Histone-lysine N-methyltransferase EZH1 (EC 2.1.1.43) (ENX-2) (Enhancer of zeste homolog 1) |
| P78549 | NTHL1 NTH1 OCTS3 | Endonuclease III-like protein 1 (hNTH1) (EC 3.2.2.-) (EC 4.2.99.18) (Bifunctional DNA N-glycosylase/DNA-(apurinic or apyrimidinic site) lyase) (DNA glycosylase/AP lyase) |
| Q12789 | GTF3C1 | General transcription factor 3C polypeptide 1 (TF3C-alpha) (TFIIIC box B-binding subunit) (Transcription factor IIIC 220 kDa subunit) (TFIIIC 220 kDa subunit) (TFIIIC220) (Transcription factor IIIC subunit alpha) |
| O14686 | KMT2D ALR MLL2 MLL4 | Histone-lysine N-methyltransferase 2D (Lysine N-methyltransferase 2D) (EC 2.1.1.43) (ALL1-related protein) (Myeloid/lymphoid or mixed-lineage leukemia protein 2) |
| Q13304 | GPR17 | Uracil nucleotide/cysteinyl leukotriene receptor (UDP/CysLT receptor) (G-protein coupled receptor 17) (P2Y-like receptor) (R12) |
| Q9UQB3 | CTNND2 NPRAP | Catenin delta-2 (Delta-catenin) (GT24) (Neural plakophilin-related ARM-repeat protein) (NPRAP) (Neurojungin) |
| P30519 | HMOX2 HO2 | Heme oxygenase 2 (HO-2) (EC 1.14.14.18) |
| O60437 | PPL KIAA0568 | Periplakin (190 kDa paraneoplastic pemphigus antigen) (195 kDa cornified envelope precursor protein) |
| Q15413 | RYR3 HBRR | Ryanodine receptor 3 (RYR-3) (RyR3) (Brain ryanodine receptor-calcium release channel) (Brain-type ryanodine receptor) (Type 3 ryanodine receptor) |
| Q13618 | CUL3 KIAA0617 | Cullin-3 (CUL-3) |
| O75691 | UTP20 DRIM | Small subunit processome component 20 homolog (Down-regulated in metastasis protein) (Novel nucleolar protein 73) (NNP73) (Protein Key-1A6) |
| O80743 | T13D8.9 | T13D8.9 protein |
| P38159 | RBMX HNRPG RBMXP1 | RNA-binding motif protein, X chromosome (Glycoprotein p43) (Heterogeneous nuclear ribonucleoprotein G) (hnRNP G) [Cleaved into: RNA-binding motif protein, X chromosome, N-terminally processed] |
| O75081 | CBFA2T3 MTG16 MTGR2 ZMYND4 | Protein CBFA2T3 (MTG8-related protein 2) (Myeloid translocation gene on chromosome 16 protein) (hMTG16) (Zinc finger MYND domain-containing protein 4) |
| O95153 | TSPOAP1 BZRAP1 KIAA0612 RBP1 RIMBP1 | Peripheral-type benzodiazepine receptor-associated protein 1 (PRAX-1) (Peripheral benzodiazepine receptor-interacting protein) (PBR-IP) (RIMS-binding protein 1) (RIM-BP1) (TSPO-associated protein 1) |
| P63267 | ACTG2 ACTA3 ACTL3 ACTSG | Actin, gamma-enteric smooth muscle (Alpha-actin-3) (Gamma-2-actin) (Smooth muscle gamma-actin) |
| P18754 | RCC1 CHC1 | Regulator of chromosome condensation (Cell cycle regulatory protein) (Chromosome condensation protein 1) |
| Q5T081 | RCC1 CHC1 hCG_27809 | CHC1 protein (Regulator of chromosome condensation 1 isoform 1) (Regulator of chromosome condensation 1, isoform CRA_b) |
| P13639 | EEF2 EF2 | Elongation factor 2 (EF-2) |
| Q16695 | HIST3H3 H3FT | Histone H3.1t (H3/t) (H3t) (H3/g) |
| A8K401 | PHB hCG_29613 | Prohibitin, isoform CRA_a (cDNA FLJ78511, highly similar to Homo sapiens prohibitin (PHB), mRNA) (cDNA, FLJ93035, Homo sapiens prohibitin (PHB), mRNA) |
| P35232 | PHB | Prohibitin |
| Q53FV0 | | Prohibitin variant (Fragment) |
| P83731 | RPL24 | 60S ribosomal protein L24 (60S ribosomal protein L30) (Large ribosomal subunit protein eL24) |
| V9HW01 | HEL-S-310 | Epididymis secretory protein Li 310 |
| A0A024RC A7 | RPLP2 hCG 1778304 | Ribosomal protein, large, P2, isoform CRA_a |
| P05387 | RPLP2 D11S2243E RPP2 | 60S acidic ribosomal protein P2 (Large ribosomal subunit protein P2) (Renal carcinoma antigen NY-REN-44) |
| P46783 | RPS10 | 40S ribosomal protein S10 (Small ribosomal subunit protein eS10) |
| P62280 | RPS11 | 40S ribosomal protein S11 (Small ribosomal subunit protein uS17) |
| P62277 | RPS13 | 40S ribosomal protein S13 (Small ribosomal subunit protein uS15) |
| P08708 | RPS17 RPS17L | 40S ribosomal protein S17 (Small ribosomal subunit protein eS17) |
| A8K517 | RPS23 hCG_38189 | Ribosomal protein S23, isoform CRA_a (cDNA FLJ77921, highly similar to Homo sapiens ribosomal protein S23 (RPS23), mRNA) (cDNA, FLJ92033, Homo sapiens ribosomal protein S23 (RPS23), mRNA) |
| P62266 | RPS23 | 40S ribosomal protein S23 (Small ribosomal subunit protein uS12) |
| P62851 | RPS25 | 40S ribosomal protein S25 (Small ribosomal subunit protein eS25) |
| B2R491 | RPS4X hCG 18634 | 40S ribosomal protein S4 |
| P62701 | RPS4X CCG2 RPS4 SCAR | 40S ribosomal protein S4, X isoform (SCR10) (Single copy abundant mRNA protein) (Small ribosomal subunit protein eS4) |
| P62241 | RPS8 OK/SW-cl.83 | 40S ribosomal protein S8 (Small ribosomal subunit protein eS8) |
| Q5JR94 | RPS8 hCG 2031852 | 40S ribosomal protein S8 |
| P12755 | SKI | Ski oncogene (Proto-oncogene c-Ski) |
| A0A1L1UH R1 | | Sperm binding protein 1a |
| B3KTS5 | | cDNA FLJ38670 fis, clone HSYRA2000190, highly similar to Voltage-dependent anion-selective channel protein 1 |
| P21796 | VDAC1 VDAC | Voltage-dependent anion-selective channel protein 1 (VDAC-1) (hVDAC1) (Outer mitochondrial membrane protein porin 1) (Plasmalemmal porin) (Porin 31HL) (Porin 31HM) |
| P25490 | YY1 INO80S | Transcriptional repressor protein YY1 (Delta transcription factor) (INO80 complex subunit S) (NF-E1) (Yin and yang 1) (YY-1) |
| Q99996 | AKAP9 AKAP350 AKAP450 KIAA0803 | A-kinase anchor protein 9 (AKAP-9) (A-kinase anchor protein 350 kDa) (AKAP 350) (hgAKAP 350) (A-kinase anchor protein 450 kDa) (AKAP 450) (AKAP 120-like protein) (Centrosome- and Golgi-localized PKN-associated protein) (CG-NAP) (Protein hyperion) (Protein kinase A-anchoring protein 9) (PRKA9) (Protein yotiao) |
| P16402 | HIST1H1D H1F3 | Histone H1.3 (Histone H1c) (Histone H1s-2) |
| Q96GY0 | ZC2HC1A C8orf70 FAM164A CGI-62 | Zinc finger C2HC domain-containing protein 1A |
| P02545 | LMNA LMN1 | Prelamin-A/C [Cleaved into: Lamin-A/C (70 kDa lamin) (Renal carcinoma antigen NY-REN-32)] |
| P20700 | LMNB1 LMN2 LMNB | Lamin-B1 |
| P14550 | AKR1A1 ALDR1 ALR | Alcohol dehydrogenase [NADP(+)] (EC 1.1.1.2) (Aldehyde reductase) (Aldo-keto reductase family 1 member A1) |
| V9HWI0 | HEL-S-165mP HEL-S-6 | Epididymis secretory protein Li 6 (Epididymis secretory sperm binding protein Li 165mP) |
| A0PJH2 | ATP5H | ATP5H protein (Fragment) |
| O75947 | ATP5H My032 | ATP synthase subunit d, mitochondrial (ATPase subunit d) |
| P19105 | MYL12A MLCB MRLC3 RLC | Myosin regulatory light chain 12A (Epididymis secretory protein Li 24) (HEL-S-24) (MLC-2B) (Myosin RLC) (Myosin regulatory light chain 2, nonsarcomeric) (Myosin regulatory light chain MRLC3) |
| A0A0G2JS 52 | | Uncharacterized protein (Fragment) |
| V9H0H3 | | Gag-Pro-Pol-Env protein |
| P17096 | HMGA1 HMGIY | High mobility group protein HMG-I/HMG-Y (HMG-I(Y)) (High mobility group AT-hook protein 1) (High mobility group protein A1) (High mobility group protein R) |
| O46577 | COX4I1 COX4 | Cytochrome c oxidase subunit 4 isoform 1, mitochondrial (Cytochrome c oxidase polypeptide IV) (Cytochrome c oxidase subunit IV isoform 1) (COX IV-1) (Fragment) |
| Q9UIG0 | BAZ1B WBSC10 WBSCR10 WBSCR9 WSTF | Tyrosine-protein kinase BAZ1B (EC 2.7.10.2) (Bromodomain adjacent to zinc finger domain protein 1B) (Williams syndrome transcription factor) (Williams-Beuren syndrome chromosomal region 10 protein) (Williams-Beuren syndrome chromosomal region 9 protein) (hWALp2) |
| Q9UHD8 | SEPT9 KIAA0991 MSF | Septin-9 (MLL septin-like fusion protein MSF-A) (MLL septin-like fusion protein) (Ovarian/Breast septin) (Ov/Br septin) (Septin D1) |
| P62270 | Rps18 | 40S ribosomal protein S18 (Ke-3) (Ke3) |
| Q561N5 | Rps18 mCG 23000 | MCG23000, isoform CRA_b (Putative uncharacterized protein) (Ribosomal protein S18) |
| Q9NWS8 | RMND1 C6orf96 | Required for meiotic nuclear division protein 1 homolog |
| P31942 | HNRNPH3 HNRPH3 | Heterogeneous nuclear ribonucleoprotein H3 (hnRNP H3) (Heterogeneous nuclear ribonucleoprotein 2H9) (hnRNP 2H9) |
| Q9NZR2 | LRP1B LRPDIT | Low-density lipoprotein receptor-related protein 1B (LRP-1B) (Low-density lipoprotein receptor-related protein-deleted in tumor) (LRP-DIT) |
| Q16891 | IMMT HMP MIC60 MINOS2 PIG4 PIG52 | MICOS complex subunit MIC60 (Cell proliferation-inducing gene 4/52 protein) (Mitochondrial inner membrane protein) (Mitofilin) (p87/89) |
| A4D1N4 | CHCHD3 hCG_2014841 tcag7.1158 | MICOS complex subunit |
| Q9NX63 | CHCHD3 MIC19 MINOS3 | MICOS complex subunit MIC19 (Coiled-coil-helix-coiled-coil-helix domain-containing protein 3) |
| Q6NTF9 | RHBDD2 RHBDL7 | Rhomboid domain-containing protein 2 |
| Q6P1M9 | ARMCX5 | Armadillo repeat-containing X-linked protein 5 |
| O00148 | DDX39A DDX39 | ATP-dependent RNA helicase DDX39A (EC 3.6.4.13) (DEAD box protein 39) (Nuclear RNA helicase URH49) |
| Q6UY01 | LRRC31 UNQ9367/PRO34 156 | Leucine-rich repeat-containing protein 31 |
| Q8IYT3 | CCDC170 C6orf97 | Coiled-coil domain-containing protein 170 |
| Q2L6I2 | ABCF1 ABC50 hCG_26012 | ABC50 protein (ATP-binding cassette, sub-family F (GCN20), member 1) (ATP-binding cassette, sub-family F (GCN20), member 1, isoform CRA_a) |
| Q8NE71 | ABCF1 ABC50 | ATP-binding cassette sub-family F member 1 (ATP-binding cassette 50) (TNF-alpha-stimulated ABC protein) |
| Q99459 | CDC5L KIAA0432 PCDC5RP | Cell division cycle 5-like protein (Cdc5-like protein) (Pombe cdc5-related protein) |
| P35580 | MYH10 | Myosin-10 (Cellular myosin heavy chain, type B) (Myosin heavy chain 10) (Myosin heavy chain, non-muscle IIb) (Non-muscle myosin heavy chain B) (NMMHC-B) (Non-muscle myosin heavy chain IIb) (NMMHC II-b) (NMMHC-IIB) |
| P50914 | RPL14 | 60S ribosomal protein L14 (CAG-ISL 7) (Large ribosomal subunit protein eL14) |
| Q9C093 | SPEF2 KIAA1770 KPL2 | Sperm flagellar protein 2 (Protein KPL2) |
| P08729 | KRT7 SCL | Keratin, type II cytoskeletal 7 (Cytokeratin-7) (CK-7) (Keratin-7) (K7) (Sarcolectin) (Type-II keratin Kb7) |
| Q9BTQ7 | | Similar to ribosomal protein L23 (Fragment) |
| Q96RT7 | TUBGCP6 GCP6 KIAA1669 | Gamma-tubulin complex component 6 (GCP-6) |
| Q5M8Q0 | Rpll5 mCG_10029 | Ribosomal protein L15 |
| Q9CZM2 | Rpl15 | 60S ribosomal protein L15 |
| Q9BS75 | KLHL20 hCG 23698 | KLHL20 protein (Kelch-like 20 (Drosophila), isoform CRA_a) |
| P82970 | HMGN5 NSBP1 | High mobility group nucleosome-binding domain-containing protein 5 (Nucleosome-binding protein 1) |
| A0A024QZ W2 | NOL7 hCG_37417 | Nucleolar protein 7, 27kDa, isoform CRA_a |
| Q9UMY1 | NOL7 C6orf90 NOP27 | Nucleolar protein 7 (Nucleolar protein of 27 kDa) |
| P62907 | Rpl10a | 60S ribosomal protein L10a |
| P78527 | PRKDC HYRC HYRC1 | DNA-dependent protein kinase catalytic subunit (DNA-PK catalytic subunit) (DNA-PKcs) (EC 2.7.11.1) (DNPK1) (p460) |
| B4E1W3 | | cDNA FLJ51732, highly similar to Peroxisomal NADH pyrophosphatase NUDT12 (EC 3.6.1.22) |
| Q9BQG2 | NUDT12 | Peroxisomal NADH pyrophosphatase NUDT12 (EC 3.6.1.22) (Nucleoside diphosphate-linked moiety X motif 12) (Nudix motif 12) |
| P46779 | RPL28 | 60S ribosomal protein L28 (Large ribosomal subunit protein eL28) |
| P22626 | HNRNPA2B1 HNRPA2B1 | Heterogeneous nuclear ribonucleoproteins A2/B1 (hnRNP A2/B1) |
| Q96Q15 | SMG1 ATX KIAA0421 LIP | Serine/threonine-protein kinase SMG1 (SMG-1) (hSMG-1) (EC 2.7.11.1) (61E3.4) (Lambda/iota protein kinase C-interacting protein) (Lambda-interacting protein) |
| A0A024R4 M0 | RPS9 hCG 2009111 | 40S ribosomal protein S9 (Ribosomal protein S9, isoform CRA_a) |
| P46781 | RPS9 | 40S ribosomal protein S9 (Small ribosomal subunit protein uS4) |
| Q96T23 | RSF1 HBXAP XAP8 | Remodeling and spacing factor 1 (Rsf-1) (HBV pX-associated protein 8) (Hepatitis B virus X-associated protein) (p325 subunit of RSF chromatin-remodeling complex) |
| P60709 | ACTB | Actin, cytoplasmic 1 (Beta-actin) [Cleaved into: Actin, cytoplasmic 1, N-terminally processed] |
| Q96RL 1 | UIMC1 RAP80 RXRIP110 | BRCA1-A complex subunit RAP80 (Receptor-associated protein 80) (Retinoid X receptor-interacting protein 110) (Ubiquitin interaction motif-containing protein 1) |
| Q96A11 | GAL3ST3 | Galactose-3-O-sulfotransferase 3 (Gal3ST-3) (EC 2.8.2.-) (Beta-galactose-3-O-sulfotransferase 3) (Gal3ST3) (Gal-beta-1, 3-GalNAc 3'-sulfotransferase 3) |
| P62847 | RPS24 | 40S ribosomal protein S24 (Small ribosomal subunit protein eS24) |
| Q9NSI6 | BRWD1 C21orf107 WDR9 | Bromodomain and WD repeat-containing protein 1 (WD repeat-containing protein 9) |
| A0A024R1 X8 | JUP hCG 1771506 | Junction plakoglobin, isoform CRA_a |
| Q96QZ7 | MAGI1 AIP3 BAIAP1 BAP1 TNRC19 | Membrane-associated guanylate kinase, WW and PDZ domain-containing protein 1 (Atrophin-1-interacting protein 3) (AIP-3) (BAI1-associated protein 1) (BAP-1) (Membrane-associated guanylate kinase inverted 1) (MAGI-1) (Trinucleotide repeat-containing gene 19 protein) (WW domain-containing protein 3) (WWP3) |
| A8K4C8 | RPL13 hCG 1723872 | 60S ribosomal protein L13 |
| P26373 | RPL13 BBC1 OK/SW-cl.46 | 60S ribosomal protein L13 (Breast basic conserved protein 1) (Large ribosomal subunit protein eL13) |
| P46019 | PHKA2 PHKLA PYK | Phosphorylase b kinase regulatory subunit alpha, liver isoform (Phosphorylase kinase alpha L subunit) |
| O60506 | SYNCRIP HNRPQ NSAP 1 | Heterogeneous nuclear ribonucleoprotein Q (hnRNP Q) (Glycine- and tyrosine-rich RNA-binding protein) (GRY-RBP) (NS1-associated protein 1) (Synaptotagmin-binding, cytoplasmic RNA-interacting protein) |
| Q96Q42 | ALS2 ALS2CR6 KIAA1563 | Alsin (Amyotrophic lateral sclerosis 2 chromosomal region candidate gene 6 protein) (Amyotrophic lateral sclerosis 2 protein) |
| Q8IYJ3 | SYTL1 SLP1 SB146 | Synaptotagmin-like protein 1 (Exophilin-7) (Protein JFC1) |
| A0A024RD H8 | RPL34 hCG 2027853 | Ribosomal protein L34, isoform CRA_a |
| P49207 | RPL34 | 60S ribosomal protein L34 (Large ribosomal subunit protein eL34) |
| Q9P2M7 | CGN KIAA1319 | Cingulin |
| Q96BT3 | CENPT C16orf56 ICEN22 | Centromere protein T (CENP-T) (Interphase centromere complex protein 22) |
| Q0VF96 | CGNL1 JACOP KIAA1749 | Cingulin-like protein 1 (Junction-associated coiled-coil protein) (Paracingulin) |
| Q96M95 | CCDC42 CCDC42A | Coiled-coil domain-containing protein 42 |
| P52597 | HNRNPF HNRPF | Heterogeneous nuclear ribonucleoprotein F (hnRNP F) (Nucleolin-like protein mcs94-1) [Cleaved into: Heterogeneous nuclear ribonucleoprotein F, N-terminally processed] |
| O96008 | TOMM40 C19orf1 PEREC1 TOM40 | Mitochondrial import receptor subunit TOM40 homolog (Protein Haymaker) (Translocase of outer membrane 40 kDa subunit homolog) (p38.5) |
| Q96BS4 | FBL | FBL protein (Putative uncharacterized protein) (Fragment) |
| Q9H501 | ESF1 ABTAP C20orf6 HDCMC28P | ESF1 homolog (ABT1-associated protein) |
| Q6PHZ2 | Camk2d Kiaa4163 | Calcium/calmodulin-dependent protein kinase type II subunit delta (CaM kinase II subunit delta) (CaMK-II subunit delta) (EC 2.7.11.17) |
| Q07020 | RPL18 | 60S ribosomal protein L18 (Large ribosomal subunit protein eL18) |
| Q8TF72 | SHROOM3 KIAA1481 SHRML MSTP013 | Protein Shroom3 (Shroom-related protein) (hShrmL) |
| Q8TE73 | DNAH5 DNAHC5 HL1 KIAA1603 | Dynein heavy chain 5, axonemal (Axonemal beta dynein heavy chain 5) (Ciliary dynein heavy chain 5) |
| O75475 | PSIP1 DFS70 LEDGF PSIP2 | PC4 and SFRS1-interacting protein (CLL-associated antigen KW-7) (Dense fine speckles 70 kDa protein) (DFS 70) (Lens epithelium-derived growth factor) (Transcriptional coactivator p75/p52) |
| E9KL44 | | Epididymis tissue sperm binding protein Li 14m |
| P40939 | HADHA HADH | Trifunctional enzyme subunit alpha, mitochondrial (78 kDa gastrin-binding protein) (TP-alpha) [Includes: Long-chain enoyl-CoA hydratase (EC 4.2.1.17); Long chain 3-hydroxyacyl-CoA dehydrogenase (EC 1.1.1.211)] |
| Q9HB09 | BCL2L12 BPR | Bcl-2-like protein 12 (Bcl2-L-12) (Bcl-2-related proline-rich protein) |
| O75367 | H2AFY MACROH2A1 | Core histone macro-H2A.1 (Histone macroH2A1) (mH2A1) (Histone H2A.y) (H2A/y) (Medulloblastoma antigen MU-MB-50.205) |
| Q8N6Z2 | MTRF1 hCG_32761 | MTRF1 protein (Mitochondrial translational release factor 1, isoform CRA_b) (Peptide chain release factor 1, mitochondrial) |
| Q8TCU4 | ALMS1 KIAA0328 | Alstrom syndrome protein 1 |
| A0JNW5 | UHRF1BP1L KIAA0701 | UHRF1-binding protein 1-like |
| O75643 | SNRNP200 ASCC3L1 HELIC2 KIAA0788 | U5 small nuclear ribonucleoprotein 200 kDa helicase (EC 3.6.4.13) (Activating signal cointegrator 1 complex subunit 3-like 1) (BRR2 homolog) (U5 snRNP-specific 200 kDa protein) (U5-200KD) |
| A7E2E1 | SMARCA4 hCG_29955 | SWI/SNF related, matrix associated, actin dependent regulator of chromatin, subfamily a, member 4 (SWI/SNF related, matrix associated, actin dependent regulator of chromatin, subfamily a, member 4, isoform CRA_a) (cDNA FLJ77531, highly similar to Homo sapiens SWI/SNF related, matrix associated, actin dependent regulator of chromatin, subfamily a, member 4 (SMARCA4), mRNA) |
| P51532 | SMARCA4 BAF190A BRG1 SNF2B SNF2L4 | Transcription activator BRG1 (EC 3.6.4.-) (ATP-dependent helicase SMARCA4) (BRG1-associated factor 190A) (BAF190A) (Mitotic growth and transcription activator) (Protein BRG-1) (Protein brahma homolog 1) (SNF2-beta) (SWI/SNF-related matrix-associated actin-dependent regulator of chromatin subfamily A member 4) |
| O00418 | EEF2K | Eukaryotic elongation factor 2 kinase (eEF-2 kinase) (eEF-2K) (EC 2.7.11.20) (Calcium/calmodulin-dependent eukaryotic elongation factor 2 kinase) |
| Q96CN4 | EVI5L | EVI5-like protein (Ecotropic viral integration site 5-like protein) |
| Q9H8V3 | ECT2 | Protein ECT2 (Epithelial cell-transforming sequence 2 oncogene) |
| Q5T3F8 | TMEM63B C6orf110 | CSC1-like protein 2 (Transmembrane protein 63B) |
| Q8NAJ6 | | cDNA FLJ35251 fis, clone PROST2003635, weakly similar to MULTIFUNCTIONAL AMINOACYL-TRNA SYNTHETASE |
| A0A0C4DG 40 | SYNE1 | Nesprin-1 |
| Q8NF91 | SYNE1 C6orf98 KIAA0796 KIAA1262 KIAA1756 MYNE1 | Nesprin-1 (Enaptin) (KASH domain-containing protein 1) (KASH1) (Myocyte nuclear envelope protein 1) (Myne-1) (Nuclear envelope spectrin repeat protein 1) (Synaptic nuclear envelope protein 1) (Syne-1) |
| Q8TDI0 | CHD5 KIAA0444 | Chromodomain-helicase-DNA-binding protein 5 (CHD-5) (EC 3.6.4.12) (ATP-dependent helicase CHD5) |
| Q9NU22 | MDN1 KIAA0301 | Midasin (MIDAS-containing protein) |
| Q8WXH0 | SYNE2 KIAA1011 NUA | Nesprin-2 (KASH domain-containing protein 2) (KASH2) (Nuclear envelope spectrin repeat protein 2) (Nucleus and actin connecting element protein) (Protein NUANCE) (Synaptic nuclear envelope protein 2) (Syne-2) |
| Q9Y277 | VDAC3 | Voltage-dependent anion-selective channel protein 3 (VDAC-3) (hVDAC3) (Outer mitochondrial membrane protein porin 3) |
| Q96QE3 | ATAD5 C17orf41 FRAG1 | ATPase family AAA domain-containing protein 5 (Chromosome fragility-associated gene 1 protein) |
| Q9BXJ9 | NAA15 GA19 NARG1 NATH TBDN100 | N-alpha-acetyltransferase 15, NatA auxiliary subunit (Gastric cancer antigen Ga19) (N-terminal acetyltransferase) (NMDA receptor-regulated protein 1) (Protein tubedown-1) (Tbdn100) |
| Q8IUE6 | HIST2H2AB | Histone H2A type 2-B |
| Q5TZA2 | CROCC KIAA0445 | Rootletin (Ciliary rootlet coiled-coil protein) |
| A0A024RA S2 | H2AFJ hCG 1639762 | Histone H2A |
| Q9BTM1 | H2AFJ | Histone H2A.J (H2a/j) |
| Q8NEN9 | PDZD8 PDZK8 | PDZ domain-containing protein 8 (Sarcoma antigen NY-SAR-84/NY-SAR-104) |
| Q14683 | SMC1A DXS423E KIAA0178 SB1.8 SMC1 SMC1L1 | Structural maintenance of chromosomes protein 1A (SMC protein 1A) (SMC-1-alpha) (SMC-1A) (Sb1.8) |
| Q68EN4 | SMC1A | SMC1A protein (Fragment) |
| Q7Z7G8 | VPS13B CHS1 COH1 KIAA0532 | Vacuolar protein sorting-associated protein 13B (Cohen syndrome protein 1) |
| Q7Z7A1 | CNTRL CEP1 CEP110 | Centriolin (Centrosomal protein 1) (Centrosomal protein of 110 kDa) (Cep110) |
| O95613 | PCNT KIAA0402 PCNT2 | Pericentrin (Kendrin) (Pericentrin-B) |
| A0A140VK 14 | | Testicular secretory protein Li 14 |
| P49448 | GLUD2 GLUDP1 | Glutamate dehydrogenase 2, mitochondrial (GDH 2) (EC 1.4.1.3) |
| Q5VTT5 | MYOM3 | Myomesin-3 (Myomesin family member 3) |
| Q7Z612 | | Acidic ribosomal phosphoprotein P1 |
| O00567 | NOP56 NOL5A | Nucleolar protein 56 (Nucleolar protein 5A) |
| Q9Y2X3 | NOP58 NOL5 NOP5 HSPC120 | Nucleolar protein 58 (Nucleolar protein 5) |
| A0A0C4DF X4 | | Uncharacterized protein (Fragment) |
| Q6ZNL4 | FLJ00279 | FLJ00279 protein (Fragment) |
| Q6ZWK7 | | cDNA FLJ16045 fis, clone CTONG2000042, weakly similar to ALPHA-2-MACROGLOBULIN |
| Q7Z388 | DPY19L4 | Probable C-mannosyltransferase DPY19L4 (EC 2.4.1.-) (Dpy-19-like protein 4) (Protein dpy-19 homolog 4) |
| Q5T9S5 | CCDC18 | Coiled-coil domain-containing protein 18 (Sarcoma antigen NY-SAR-24) |
| Q6ZV73 | FGD6 KIAA1362 ZFYVE24 | FYVE, RhoGEF and PH domain-containing protein 6 (Zinc finger FYVE domain-containing protein 24) |
| P25705 | ATP5A1 ATP5A ATP5AL2 ATPM | ATP synthase subunit alpha, mitochondrial |
| P42285 | SKIV2L2 DOB 1 KIAA0052 Mtr4 | Superkiller viralicidic activity 2-like 2 (EC 3.6.4.13) (ATP-dependent RNA helicase DOB1) (ATP-dependent RNA helicase SKIV2L2) (TRAMP-like complex helicase) |
| Q00325 | SLC25A3 PHC OK/SW-cl.48 | Phosphate carrier protein, mitochondrial (Phosphate transport protein) (PTP) (Solute carrier family 25 member 3) |
| P62753 | RPS6 OK/SW-cl.2 | 40S ribosomal protein S6 (Phosphoprotein NP33) (Small ribosomal subunit protein eS6) |
| Q9BW34 | EEF1D | EEF1D protein (Fragment) |
| Q5K651 | SAMD9 C7orf5 DRIF1 KIAA2004 OEF1 | Sterile alpha motif domain-containing protein 9 (SAM domain-containing protein 9) |
| Q6W6M6 | | Antigen MLAA-44 |
| Q5T0F9 | CC2D1B KIAA1836 | Coiled-coil and C2 domain-containing protein 1B (Five prime repressor element under dual repression-binding protein 2) (FRE under dual repression-binding protein 2) (Freud-2) |
| P26641 | EEF1G EF1G PRO1608 | Elongation factor 1-gamma (EF-1-gamma) (eEF-1B gamma) |
| Q00839 | HNRNPU HNRPU SAFA U21.1 | Heterogeneous nuclear ribonucleoprotein U (hnRNP U) (Scaffold attachment factor A) (SAF-A) (p120) (pp120) |
| Q9Y4C4 | MFHAS1 MASL1 | Malignant fibrous histiocytoma-amplified sequence 1 (Malignant fibrous histiocytoma-amplified sequence with leucine-rich tandem repeats 1) |
| P16050 | ALOX15 LOG15 | Arachidonate 15-lipoxygenase (15-LOX) (15-LOX-1) (EC 1.13.11.33) (12/15-lipoxygenase) (Arachidonate 12-lipoxygenase, leukocyte-type) (12-LOX) (EC 1.13.11.31) (Arachidonate omega-6 lipoxygenase) |
| P16383 | GCFC2 C2orf3 GCF TCF9 | GC-rich sequence DNA-binding factor 2 (GC-rich sequence DNA-binding factor) (Transcription factor 9) (TCF-9) |
| P36578 | RPL4 RPL1 | 60S ribosomal protein L4 (60S ribosomal protein L1) (Large ribosomal subunit protein uL4) |
| O76081 | RGS20 RGSZ1 ZGAP1 | Regulator of G-protein signaling 20 (RGS20) (Gz-selective GTPase-activating protein) (G(z)GAP) (Gz-GAP) (Regulator of G-protein signaling Z1) (Regulator of Gz-selective protein signaling 1) |
| Q9Y6N9 | USH1C AIE75 | Harmonin (Antigen NY-CO-38/NY-CO-37) (Autoimmune enteropathy-related antigen AIE-75) (Protein PDZ-73) (Renal carcinoma antigen NY-REN-3) (Usher syndrome type-1C protein) |
| Q15149 | PLEC PLEC1 | Plectin (PCN) (PLTN) (Hemidesmosomal protein 1) (HD1) (Plectin-1) |
| O60333 | KIF1B KIAA0591 KIAA1448 | Kinesin-like protein KIF1B (Klp) |
| O60462 | NRP2 VEGF165R2 | Neuropilin-2 (Vascular endothelial cell growth factor 165 receptor 2) |
| Q7Z3T9 | DKFZp686J1169 | Neuropilin |
| Q5THJ4 | VPS13D KIAA0453 | Vacuolar protein sorting-associated protein 13D |
| Q9NRZ9 | HELLS PASG SMARCA6 Nbla10143 | Lymphoid-specific helicase (EC 3.6.4.-) (Proliferation-associated SNF2-like protein) (SWI/SNF2-related matrix-associated actin-dependent regulator of chromatin subfamily A member 6) |
| Q96A08 | HIST1H2BA TSH2B | Histone H2B type 1-A (Histone H2B, testis) (TSH2B.1) (hTSH2B) (Testis-specific histone H2B) |
| Q6UB99 | ANKRD11 ANCO1 | Ankyrin repeat domain-containing protein 11 (Ankyrin repeat-containing cofactor 1) |
| I6L9F7 | HIST1H2BM | Histone H2B (Fragment) |
| P02538 | KRT6A K6A KRT6D | Keratin, type II cytoskeletal 6A (Cytokeratin-6A) (CK-6A) (Cytokeratin-6D) (CK-6D) (Keratin-6A) (K6A) (Type-II keratin Kb6) (allergen Hom s 5) |
| Q6KC79 | NIPBL IDN3 | Nipped-B-like protein (Delangin) (SCC2 homolog) |
| Q8NBU5 | ATAD1 FNP001 | ATPase family AAA domain-containing protein 1 (EC 3.6.1.3) (Thorase) |
| E5KLM2 | | Mitochondrial dynamin-like 120 kDa protein |
| Q15772 | SPEG APEG1 KIAA1297 | Striated muscle preferentially expressed protein kinase (EC 2.7.11.1) (Aortic preferentially expressed protein 1) (APEG-1) |
| O14490 | DLGAP1 DAP1 GKAP | Disks large-associated protein 1 (DAP-1) (Guanylate kinase-associated protein) (hGKAP) (PSD-95/SAP90-binding protein 1) (SAP90/PSD-95-associated protein 1) (SAPAP1) |
| Q5JSL3 | DOCK11 ZIZ2 | Dedicator of cytokinesis protein 11 (Activated Cdc42-associated guanine nucleotide exchange factor) (ACG) (Zizimin-2) |
| Q5VU43 | PDE4DIP CMYA2 KIAA0454 KIAA0477 MMGL | Myomegalin (Cardiomyopathy-associated protein 2) (Phosphodiesterase 4D-interacting protein) |
| Q658X5 | DKFZp666F 1010 | Putative uncharacterized protein DKFZp666F1010 (Fragment) |
| Q658W4 | DKFZp666M0710 | Putative uncharacterized protein DKFZp666M0710 (Fragment) |
| Q63HR1 | DKFZp686P17171 | Putative uncharacterized protein DKFZp686P17171 |
| Q5VWT5 | ARAP C1orf168 | Activation-dependent, raft-recruited ADAP-like phosphoprotein |
| Q92614 | MYO18A CD245 KIAA0216 MYSPDZ | Unconventional myosin-XVIIIa (Molecule associated with JAK3 N-terminus) (MAJN) (Myosin containing a PDZ domain) (Surfactant protein receptor SP-R210) (SP-R210) |
| A0A024R4 A0 | NCL hCG_33980 | Nucleolin, isoform CRA_b |
| B3KM80 | NCL hCG_33980 | Nucleolin, isoform CRA_c (cDNA FLJ10452 fis, clone NT2RP 1000966, highly similar to NUCLEOLIN) |
| P19338 | NCL | Nucleolin (Protein C23) |
| P35527 | KRT9 | Keratin, type I cytoskeletal 9 (Cytokeratin-9) (CK-9) (Keratin-9) (K9) |
| Q5T655 | CFAP58 C10orf80 CCDC147 | Cilia- and flagella-associated protein 58 (Coiled-coil domain-containing protein 147) |
| Q5TAX3 | ZCCHC11 KIAA0191 TUT4 | Terminal uridylyltransferase 4 (TUTase 4) (EC 2.7.7.52) (Zinc finger CCHC domain-containing protein 11) |
| Q9Y6I7 | WSB1 SWIP1 | WD repeat and SOCS box-containing protein 1 (WSB-1) (SOCS box-containing WD protein SWiP-1) |
| Q9HC77 | CENPJ CPAP LAP LIP1 | Centromere protein J (CENP-J) (Centrosomal P4.1-associated protein) (LAG-3-associated protein) (LYST-interacting protein 1) |
| Q5H8C1 | FREM1 C9orf143 C9orf145 C9orf154 | FRAS1-related extracellular matrix protein 1 (Protein QBRICK) |
| Q8N5G2 | TMEM57 | Macoilin (Transmembrane protein 57) |
| Q58F05 | NARG1 | NARG1 protein (Fragment) |
| Q59HE3 | | Calpastatin isoform a variant (Fragment) |
| Q59GX9 | | Ribosomal protein L5 variant (Fragment) |
| Q59FF1 | | Insulin-like growth factor binding protein 2 variant (Fragment) |
| O75116 | ROCK2 KIAA0619 | Rho-associated protein kinase 2 (EC 2.7.11.1) (Rho kinase 2) (Rho-associated, coiled-coil-containing protein kinase 2) (Rho-associated, coiled-coil-containing protein kinase II) (ROCK-II) (p164 ROCK-2) |
| Q53HW2 | | 60S acidic ribosomal protein P0 (Fragment) |
| Q53HR5 | | Elongation factor 1-alpha (Fragment) |
| P14136 | GFAP | Glial fibrillary acidic protein (GFAP) |
| Q562R1 | ACTBL2 | Beta-actin-like protein 2 (Kappa-actin) |
| A0A024R2 G2 | FANCD2 hCG_1811443 | Fanconi anemia, complementation group D2, isoform CRA_b |
| Q9BXW9 | FANCD2 FACD | Fanconi anemia group D2 protein (Protein FACD2) |
| Q86XH1 | IQCA1 IQCA | IQ and AAA domain-containing protein 1 |
| A1XBS5 | FAM92A FAM92A1 | Protein FAM92A |
| Q9P273 | TENM3 KIAA1455 ODZ3 TNM3 | Teneurin-3 (Ten-3) (Protein Odd Oz/ten-m homolog 3) (Tenascin-M3) (Ten-m3) (Teneurin transmembrane protein 3) |
| Q9P2K1 | CC2D2A KIAA1345 | Coiled-coil and C2 domain-containing protein 2A |
| Q96BT1 | C3orf49 | Putative uncharacterized protein C3orf49 |
| P09651 | HNRNPA1 HNRPA1 | Heterogeneous nuclear ribonucleoprotein A1 (hnRNP A1) (Helix-destabilizing protein) (Single-strand RNA-binding protein) (hnRNP core protein A1) [Cleaved into: Heterogeneous nuclear ribonucleoprotein A1, N-terminally processed] |
| Q9P225 | DNAH2 DNAHC2 DNHD3 KIAA1503 | Dynein heavy chain 2, axonemal (Axonemal beta dynein heavy chain 2) (Ciliary dynein heavy chain 2) (Dynein heavy chain domain-containing protein 3) |
| Q4KM60 | Rpl10a Serpina6 | Ribosomal protein (Fragment) |
| Q32Q62 | RSL1D1 | RSL1D1 protein (Fragment) |
| Q9H611 | PIF1 C15orf20 | ATP-dependent DNA helicase PIF1 (EC 3.6.4.12) (DNA repair and recombination helicase PIF1) (PIF1/RRM3 DNA helicase-like protein) |
| Q86Y46 | KRT73 K6IRS3 KB36 KRT6IRS3 | Keratin, type II cytoskeletal 73 (Cytokeratin-73) (CK-73) (Keratin-73) (K73) (Type II inner root sheath-specific keratin-K6irs3) (Type-II keratin Kb36) |
| Q0QEN7 | ATP5B | ATP synthase subunit beta (EC 3.6.3.14) (Fragment) |
| B3KU66 | | cDNA FLJ39263 fis, clone OCBBF2009571, highly similar to ATP-dependent RNA helicase A (EC 3.6.1.-) |
| Q08211 | DHX9 DDX9 LKP NDH2 | ATP-dependent RNA helicase A (RHA) (EC 3.6.4.13) (DEAH box protein 9) (Leukophysin) (LKP) (Nuclear DNA helicase II) (NDH II) |
| O15078 | CEP290 BBS14 KIAA0373 NPHP6 | Centrosomal protein of 290 kDa (Cep290) (Bardet-Biedl syndrome 14 protein) (Cancer/testis antigen 87) (CT87) (Nephrocystin-6) (Tumor antigen se2-2) |
| Q05BJ6 | CEP290 | CEP290 protein |
| Q92538 | GBF1 KIAA0248 | Golgi-specific brefeldin A-resistance guanine nucleotide exchange factor 1 (BFA-resistant GEF 1) |
| Q4G0J3 | LARP7 HDCMA18P | La-related protein 7 (La ribonucleoprotein domain family member 7) (P-TEFb-interaction protein for 7SK stability) (PIP7S) |
| Q15397 | PUM3 cPERP-C KIAA0020 PUF-A XTP5 | Pumilio homolog 3 (HBV X-transactivated gene 5 protein) (HBV XAg-transactivated protein 5) (Minor histocompatibility antigen HA-8) (HLA-HA8) |
| Q7RTY7 | OVCH1 | Ovochymase-1 (EC 3.4.21.-) |
| Q5SPB7 | ino80 si:ch211-244p18.3 | INO80 complex subunit |
| Q9Y3V2 | RWDD3 RSUME | RWD domain-containing protein 3 (RWD domain-containing sumoylation enhancer) (RSUME) |
| Q9HCR9 | PDE11A | Dual 3',5'-cyclic-AMP and -GMP phosphodiesterase 11A (EC 3.1.4.35) (EC 3.1.4.53) (cAMP and cGMP phosphodiesterase 11A) |
| Q9NR48 | ASH1L KIAA1420 KMT2H | Histone-lysine N-methyltransferase ASH1L (EC 2.1.1.43) (ASH1-like protein) (huASH1) (Absent small and homeotic disks protein 1 homolog) (Lysine N-methyltransferase 2H) |
| Q09428 | ABCC8 HRINS SUR SUR1 | ATP-binding cassette sub-family C member 8 (Sulfonylurea receptor 1) |
| Q5JU67 | CFAP157 C9orf117 | Cilia- and flagella-associated protein 157 |
| D3DR32 | MPHOSPH1 hCG 23744 | M-phase phosphoprotein 1, isoform CRA_a |
| G5E9G0 | RPL3 ASC-1 hCG 2015191 | 60S ribosomal protein L3 (Ribosomal protein L3, isoform CRA e) |
| D3DS91 | AKAP6 hCG 1812123 | A kinase (PRKA) anchor protein 6, isoform CRA_b |
| A0A0A7M1 X5 | LMNB2 hCG 2004338 | Lamin B2, isoform CRA_b (Lamin B3) |
| A0A024R5 M9 | NUMA1 hCG 2017131 | Nuclear mitotic apparatus protein 1, isoform CRA_a |
| Q4G0X9 | CCDC40 KIAA1640 | Coiled-coil domain-containing protein 40 |
| D3DTT5 | TBKBP 1 hCG 1813987 | TBK1 binding protein 1, isoform CRA_a |
| G5E972 | TMPO hCG 2015322 | Lamina-associated polypeptide 2, isoforms beta/gamma (Thymopoietin, isoform CRA d) |
| D6W5D1 | KIAA1212 hCG 1817741 | KIAA1212, isoform CRA_a |
| U3KQK0 | HIST1H2BN hCG 1743059 | Histone H2B |
| D6RGI3 | SEPT11 hCG_24410 | Septin 11, isoform CRA_b (Septin-11) |
| B4DDB6 | HNRPA3 hCG_2005824 | Heterogeneous nuclear ribonucleoprotein A3, isoform CRA_a (cDNA FLJ52659, highly similar to Heterogeneous nuclear ribonucleoprotein A3) (cDNA, FLJ79333, highly similar to Heterogeneous nuclear ribonucleoprotein A3) |
| Q8TE76 | MORC4 ZCW4 ZCWCC2 | MORC family CW-type zinc finger protein 4 (Zinc finger CW-type coiled-coil domain protein 2) (Zinc finger CW-type domain protein 4) |
| Q8NCM8 | DYNC2H1 DHC1B DHC2 | Cytoplasmic dynein 2 heavy chain 1 (Cytoplasmic dynein 2 heavy chain) (Dynein cytoplasmic heavy |
| | DNCH2 DYH1B KIAA1997 | chain 2) (Dynein heavy chain 11) (hDHC11) (Dynein heavy chain isotype 1B) |
| Q6PIF6 | MYO7B | Unconventional myosin-VIIb |
| Q8NB66 | UNC13C | Protein unc-13 homolog C (Munc13-3) |
| A0A1U9X7 W7 | | HSPA1L |
| P34931 | HSPA1L | Heat shock 70 kDa protein 1-like (Heat shock 70 kDa protein 1L) (Heat shock 70 kDa protein 1-Hom) (HSP70-Hom) |
| A4D0S4 | LAMB4 | Laminin subunit beta-4 (Laminin beta-1-related protein) |
| Q8N309 | LRRC43 | Leucine-rich repeat-containing protein 43 |
| Q8TDW7 | FAT3 CDHF15 KIAA1989 | Protocadherin Fat 3 (hFat3) (Cadherin family member 15) (FAT tumor suppressor homolog 3) |
| A5WVL9 | dapk1 si:ch211-66i11.1 | Death-associated protein kinase (Death-associated protein kinase 1) |
| P05141 | SLC25A5 ANT2 | ADP/ATP translocase 2 (ADP,ATP carrier protein 2) (ADP,ATP carrier protein, fibroblast isoform) (Adenine nucleotide translocator 2) (ANT 2) (Solute carrier family 25 member 5) [Cleaved into: ADP/ATP translocase 2, N-terminally processed] |
| Q6NVC0 | SLC25A5 | SLC25A5 protein (Fragment) |
| P12236 | SLC25A6 ANT3 CDABP0051 | ADP/ATP translocase 3 (ADP,ATP carrier protein 3) (ADP,ATP carrier protein, isoform T2) (ANT 2) (Adenine nucleotide translocator 3) (ANT 3) (Solute carrier family 25 member 6) [Cleaved into: ADP/ATP translocase 3, N-terminally processed] |
| Q6I9V5 | SLC25A6 hCG_1746794 | SLC25A6 protein (Solute carrier family 25 (Mitochondrial carrier adenine nucleotide translocator), member 6) (cDNA, FLJ92654, highly similar to Homo sapiens solute carrier family 25 (mitochondrial carrier; adenine nucleotide translocator), member 6 (SLC25A6), mRNA) |
| Q0VGD6 | HNRPR | HNRPR protein (Fragment) |
| A0A024R3 T8 | PARP1 hCG_14746 | Poly [ADP-ribose] polymerase (PARP) (EC 2.4.2.30) |
| P09874 | PARP1 ADPRT PPOL | Poly [ADP-ribose] polymerase 1 (PARP-1) (EC 2.4.2.30) (ADP-ribosyltransferase diphtheria toxin-like 1) (ARTD1) (NAD(+) ADP-ribosyltransferase 1) (ADPRT 1) (Poly[ADP-ribose] synthase 1) |
| Q8IVF2 | AHNAK2 C14orf78 KIAA2019 | Protein AHNAK2 |
| Q9BQG0 | MYBBP1A P160 | Myb-binding protein 1A |
| A6PVS8 | LRRIQ3 LRRC44 | Leucine-rich repeat and IQ domain-containing protein 3 (Leucine-rich repeat-containing protein 44) |
| A8K6K6 | | cDNA FLJ76880 |
| A8K2G7 | | cDNA FLJ76071, highly similar to Homo sapiens filamin A interacting protein 1 (FILIP1), mRNA |
| B0AZQ4 | | Structural maintenance of chromosomes protein |
| Q9P1Z9 | CCDC180 C9orf174 KIAA1529 | Coiled-coil domain-containing protein 180 |
| Q9UFH2 | DNAH17 DNAHL1 DNEL2 | Dynein heavy chain 17, axonemal (Axonemal beta dynein heavy chain 17) (Axonemal dynein heavy chain-like protein 1) (Ciliary dynein heavy chain 17) (Ciliary dynein heavy chain-like protein 1) (Dynein light chain 2, axonemal) |
| B2R5B3 | | Histone H2A |
| B2RAM8 | | cDNA, FLJ95007, highly similar to Homo sapiens BRCA1 associated RING domain 1 (BARD1), mRNA |
| Q68CZ1 | RPGRIP1L FTM KIAA1005 NPHP8 | Protein fantom (Nephrocystin-8) (RPGR-interacting protein 1-like protein) (RPGRIP1-like protein) |
| Q2QL34 | MPV17L | Mpv17-like protein (M-LP homolog) (M-LPH) |
| Q13948 | CUX1 CUTL1 | Protein CASP |
| B3KX72 | | cDNA FLJ44920 fis, clone BRAMY3011501, highly similar to Heterogeneous nuclear ribonucleoprotein U |
| Q9NVI7 | ATAD3A | ATPase family AAA domain-containing protein 3A |
| B3KS36 | | cDNA FLJ35376 fis, clone SKMUS2004044, highly similar to Homo sapiens ribosomal protein L3 (RPL3), transcript variant 2, mRNA |
| D7EZH4 | | SNF2LT |
| Q9C0G6 | DNAH6 DNAHC6 DNHL1 HL2 KIAA1697 | Dynein heavy chain 6, axonemal (Axonemal beta dynein heavy chain 6) (Ciliary dynein heavy chain 6) |
| O60524 | NEMF SDCCAG1 | Nuclear export mediator factor NEMF (Antigen NY-CO-1) (Serologically defined colon cancer antigen 1) |
| B4DWU6 | | cDNA FLJ51361, highly similar to Keratin, type II cytoskeletal 6A |
| B4DXG0 | | cDNA FLJ57651, highly similar to Ketosamine-3-kinase (EC 2.7.1.-) |
| B4DGN6 | | cDNA FLJ50007 |
| B4DXQ8 | | cDNA FLJ52940, highly similar to Mortality factor 4-like protein 2 |
| Q7L099 | RUFY3 KIAA0871 | Protein RUFY3 (RUN and FYVE domain-containing protein 3) (Rap2-interacting protein x) (RIPx) (Single axon-regulated protein) (Singar) |
| Q9C099 | LRRCC1 CLERC KIAA1764 | Leucine-rich repeat and coiled-coil domain-containing protein 1 (Centrosomal leucine-rich repeat and coiled-coil domain-containing protein) |
| B4DYY8 | | cDNA FLJ60374 |
| Q14439 | GPR176 | G-protein coupled receptor 176 (HB-954) |
| B4DZM3 | | cDNA FLJ61500, highly similar to NNP-1 protein |
| P62318 | SNRPD3 | Small nuclear ribonucleoprotein Sm D3 (Sm-D3) (snRNP core protein D3) |
| B4E1T1 | | cDNA FLJ54081, highly similar to Keratin, type II cytoskeletal 5 |
| B4DLB1 | | cDNA FLJ58017, moderately similar to Treacle protein |
| Q8TC59 | PIWIL2 HILI | Piwi-like protein 2 (EC 3.1.26.-) (Cancer/testis antigen 80) (CT80) |
| Q16513 | PKN2 PRK2 PRKCL2 | Serine/threonine-protein kinase N2 (EC 2.7.11.13) (PKN gamma) (Protein kinase C-like 2) (Protein-kinase C-related kinase 2) |
| O75923 | DYSF FER1L1 | Dysferlin (Dystrophy-associated fer-1-like protein) (Fer-1-like protein 1) |
| Q5RF89 | DKFZp469P0721 | Putative uncharacterized protein DKFZp469P0721 |
| Q9UBN4 | TRPC4 | Short transient receptor potential channel 4 (TrpC4) (Trp-related protein 4) (hTrp-4) (hTrp4) |
| P62826 | RAN ARA24 OK/SW-cl.81 | GTP-binding nuclear protein Ran (Androgen receptor-associated protein 24) (GTPase Ran) (Ras-like protein TC4) (Ras-related nuclear protein) |
| Q6NTA2 | HNRNPL | HNRNPL protein (Fragment) |
| B4DPC0 | | cDNA FLJ52713, moderately similar to Mus musculus leucine rich repeat (in FLII) interacting protein 1 (Lrrfip1), mRNA |
| B7Z2C5 | | cDNA FLJ50492, highly similar to Cyclin-dependent kinase-like 3 (EC 2.7.11.22) |
| Q86TI0 | TBC1D1 KIAA1108 | TBC1 domain family member 1 |
| Q15233 | NONO NRB54 | Non-POU domain-containing octamer-binding protein (NonO protein) (54 kDa nuclear RNA- and DNA-binding protein) (55 kDa nuclear protein) (DNA-binding p52/p100 complex, 52 kDa subunit) (NMT55) (p54(nrb)) (p54nrb) |
| B7Z4E3 | RPL31 | 60S ribosomal protein L31 (cDNA FLJ58908, highly similar to 60S ribosomal protein L31) |
| B7Z7K9 | | cDNA FLJ51382 |
| Q92833 | JARID2 JMJ | Protein Jumonji (Jumonji/ARID domain-containing protein 2) |
| Q8N398 | VWA5B2 | von Willebrand factor A domain-containing protein 5B2 |
| Q9BVH8 | VWA5B2 | VWA5B2 protein (Fragment) |
| Q6ZU80 | CEP128 C14orf145 C14orf61 | Centrosomal protein of 128 kDa (Cep128) |
| P46013 | MKI67 | Proliferation marker protein Ki-67 (Antigen identified by monoclonal antibody Ki-67) (Antigen KI-67) (Antigen Ki67) |
| A2A547 | Rpl19 | Ribosomal protein L19 |
| E4W6B6 | RPL27 | RPL27/NME2 fusion protein (Fragment) |
| O15050 | TRANK1 KIAA0342 LBA1 | TPR and ankyrin repeat-containing protein 1 (Lupus brain antigen 1 homolog) |
| B3KQL5 | | cDNA FLJ90678 fis, clone PLACE1005736, highly similar to Pleckstrin homology domain-containing family A member 1 |
| Q9HB21 | PLEKHA1 TAPP1 | Pleckstrin homology domain-containing family A member 1 (PH domain-containing family A member 1) (Tandem PH domain-containing protein 1) (TAPP-1) |
| O60264 | SMARCA5 SNF2H WCRF135 | SWI/SNF-related matrix-associated actin-dependent regulator of chromatin subfamily A member 5 (SWI/SNF-related matrix-associated actin-dependent regulator of chromatin A5) (EC 3.6.4.-) (Sucrose nonfermenting protein 2 homolog) (hSNF2H) |
| Q14789 | GOLGB1 | Golgin subfamily B member 1 (372 kDa Golgi complex-associated protein) (GCP372) (Giantin) (Macrogolgin) |
| A0A087W UK2 | HNRNPDL HNRPDL hCG 22986 | Heterogeneous nuclear ribonucleoprotein D-like (Heterogeneous nuclear ribonucleoprotein D-like, isoform CRA_b) |
| O14979 | HNRNPDL HNRPDL JKTBP | Heterogeneous nuclear ribonucleoprotein D-like (hnRNP D-like) (hnRNP DL) (AU-rich element RNA-binding factor) (JKT41-binding protein) (Protein laAUF1) |

In one aspect, the active agent may be selected from one or more compounds as listed in Table 2.

**Table 2. Compounds that inhibit proteins that inhibit nucleic acid delivery vehicle uptake.**

| **Compound name** | **Structure** | **Source** | **CAS registry** | **Pubchem ID** | **PMID** |
|---|---|---|---|---|---|
| Geldanamycin and derivative Alvespimycin | 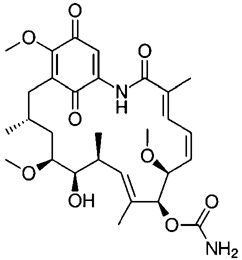 | **Multiple,** Wutech | 30562-34-6 | 5288382 | 1551101 2656616 |
| | | Acorn PharmaTech Product List | | | |
| | | ZINC | | | |
| | | **OWNED by,** Novartis | | | |
| Entasobulin | 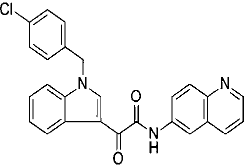 | **Multiple,** ZINC | 501921-61-5 | 10203597 | |
| | | MedChemexp ress MCE ChemScene | | | |
| Androstanolone/ Dihydrotestostero ne | 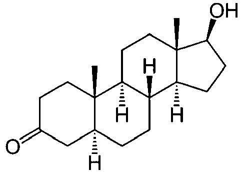 | **Multiple,** Sigma-Aldrich | 12040-51-6, 28801-96-9, 29873-50-5, 521-18-6, 571-22-2 | 10635, 15 | 1660453 8 2003561 5 2042747 6 |
| | | Key Organics/BIO NET 1717 CheMall Corporation **OWNED by,** | | | |
| Spermine (Spermine-RX-11, CKII activation) | 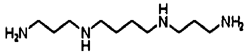 | **Multiple,** Finetech Industry Limited AK Scientific, Inc. (AKSCI) Sigma-Aldrich | 71-44-3 | 1103 | 2696287 3 6534776 2042747 6 3878 |
| Cortisone | 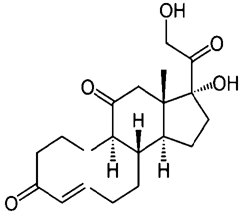 | **Multiple,** Ambinter | 53-06-5 | 222786 | 24 2785665 5 |
| | | LGC Standards | | | |
| | | AKos Consulting & Solutions | | | |
| Quercetin | 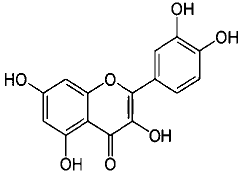 | **Multiple,** BePharm Ltd. | 117-39-5, 6151-25-3, 7255-55-2, 73123-10-1, 74893-81-5 | 5280343 | 2857457 4 |
| | | Ambinter | | | |
| | | TimTec | | | |
| Acetohexamide (Acetohexamide-RX013, ABCC8 activation) | 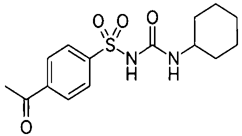 | **Multiple,** TargetMol | 8054-32-8, 968-81-0 | 1989 | 21249 2264568 9 |
| | | Boc Sciences | | | |
| | | Angene Chemical **OWNED by,** Watson Lilly | | | |
| Resveratrol | 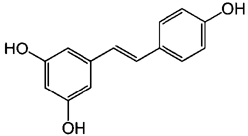 | **Multiple,** 1717 CheMall Corporation | 501-36-0 | 445154 | 7497631 2849973 2 2840697 4 |
| | | ApexBio Technology | | | |
| | | Selleckchem | | | |
| | | **OWNED by,** Home Aide Diagnostics, Inc. | | | |
| Doxorubicin (Doxorubicin-RX012, modulator of multiple cytosolic interaction) | 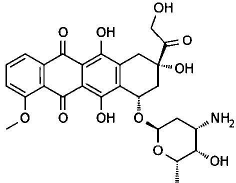 | **Multiple,** AbovChem LLC Alsachim | 23214-92-8, 25316-40-9 | 31703 | 3405 14644 2865737 2 2871837 0 |
| | | ABBLIS Chemicals **OWNED by,** Pfizer | | | |
| Ruxolitinib (Ruxolitinib-RX008, JAK1 inhibition) | 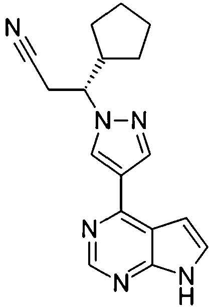 | **Multiple,** BePharm Ltd. | 1092939-17-7 | 25126798 | 1938567 2 1946827 5 2852087 1 |
| | | AvaChem Scientific Active Biopharma | | | |
| | | **OWNED by,** Novartis | | | |
| Roscovitine/ Seliciclib (Roscovitine-RX001, CDK1 inhibition) | 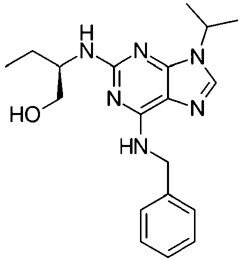 | **Multiple,** Tocris Bioscience abcr GmbH | 186692-44-4 | 5097 | 2696287 3 9046330 2069273 7 |
| | | Boc Sciences | | | |
| | | **OWNED by,** Cyclacel Pharmaceutica ls Inc. | | | |
| Sildenafil (Sildenafil Citrate-RX014, PDE11A inhibition) | 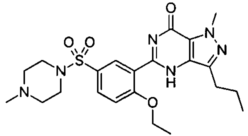 | **Multiple,** OXCHEM CORPORATI ON | 139755-83-2, 171599-83-0 | 5212 | 2865226 2 2853553 6 2864007 7 |
| | | MolPort | | | |
| | | Vitas-M Laboratory | | | |
| | | **OWNED by,** Pfizer | | | |
| | | Actavis Pharma Company | | | |
| | | | | | |
| Teniposide / Vumon | 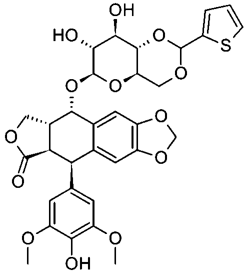 | **Multiple,** AK Scientific, Inc. | 23362-13-2, 29767-20-2, 31514-29-1, 35317-44-3 | 34698 | 2691615 0 2658361 1 2277170 6 |
| | | AbovChem LLC | | | |
| | | Boc Sciences | | | |
| | | **OWNED by,** WG Critical Care, LLC | | | |
| | | Bristol Myers Squibb | | | |

**Description of Agents in Table 2.** Geldanamycin is a benzoquinone ansamycin that binds to the heat shock protein Hsp90 and activates a heat shock response in mammalian cells. Entasobulin is the first anticancer drug in development involving two mechanisms of action, tubulin and topoisomerase II inhibition. Entasobulin expresses different modes of action such as, pro-apoptotic and anti-angiogenic properties. Dihydrotestosterone (DHT) (INN: androstanolone) is a biologically active metabolite of the hormone testosterone, formed primarily in the prostate gland, testes, hair follicles, and adrenal glands by the enzyme 5-alpha-reductase by means of reducing the alpha 4, 5 double-bond. Dihydrotestosterone belongs to the class of compounds called androgens, also commonly called androgenic hormones or testoids. DHT is thought to be approximately 30 times more potent than testosterone because of increased affinity to the androgen receptor. Spermine is a polyamine involved in cellular metabolism found in all eukaryotic cells. The precursor for synthesis of spermine is the amino acid omithine. It is found in a wide variety of organisms and tissues and is an essential growth factor in some bacteria. It is found as a polycation at physiological pH. Spermine is associated with nucleic acids and is thought to stabilize helical structure, particularly in viruses. Cortisone is a Corticosteroid. The mechanism of action of cortisone is as a Corticosteroid Hormone Receptor Agonist. Quercetin is a flavonoid and more specifically a flavonol and represents 60% of the total dietary flavonols intake. The term flavonoid comprises several thousand plant derived compounds sharing a common skeleton of phenyl-chromane. This basic structure allows a multitude of substitution patterns leading to several flavonoid subclasses such as flavonols, flavones, flavanones, catechins, anthocyanidins, isoflavones, dihydroflavonols and chalcones. The first generation sulfonylureas include acetohexamide, chlorpropamide, tolazamide and tolbutamide, oral hypoglycemic agents that are used in therapy of type 2 diabetes. Resveratrol (3,5,4'-trihydroxystilbene) is a polyphenolic phytoalexin. It is a stilbenoid, a derivate of stilbene, and is produced in plants with the help of the enzyme stilbene synthase. It exists as two structural isomers: cis-(Z) and trans-(E), with the trans-isomer shown in the top image. The trans- form can undergo isomerization to the cis- form when heated or exposed to ultraviolet irradiation. In a 2004 issue of Science, Dr. Sinclair of Harvard University said resveratrol is not an easy molecule to protect from oxidation. It has been claimed that it is readily degraded by exposure to light, heat, and oxygen. However, studies find that Trans-resveratrol undergoes negligible oxidation in normal atmosphere at room temperature. Doxorubicin is a drug used in cancer chemotherapy. It is an anthracycline antibiotic, closely related to the natural product daunomycin, and like all anthracyclines it intercalates DNA. It is commonly used in the treatment of a wide range of cancers, including hematological malignancies, many types of carcinoma, and soft tissue sarcomas. The drug is administered in the form of hydrochloride salt intravenously. It may be sold under the brand names Adriamycin PFS, Adriamycin RDF, or Rubex. It is photosensitive and it is often covered by an aluminum bag to prevent light from affecting it. Ruxolitinib (INCB018424) is a selective oral JAK1/JAK2 inhibitor. This agent has the potential to modulate two important kinases that may play a role in myeloproliferative neoplasms, including primary myelofibrosis. Roscovitine is a Potent and Selective Inhibitor of the Cyclin-Dependent Kinases cdc2, cdk2 and cdk5. Sildenafil is a selective PDE5 inhibitor that is used to treat erectile dysfunction and pulmonary arterial hypertension. Teniposide/Vumon is a semisynthetic derivative of podophyllotoxin with antineoplastic activity. Teniposide forms a ternary complex with the enzyme topoisomerase II and DNA, resulting in dose-dependent single- and double-stranded breaks in DNA, DNA: protein cross-links, inhibition of DNA strand religation, and cytotoxicity. This agent acts in the late S or early G phase of the cell cycle.

**Table 3. Compounds that inhibit proteins that inhibit nucleic acid delivery vehicle uptake**

| **#** | **Drug** | **Gene Symbol** | **Target** | **Effect** | **Pubmed** |
|---|---|---|---|---|---|
| 1 | Entasobulin intracellular | TOP2B | TOP2 beta | Inhibition | |
| 2 | Memantine extracellular region | GRIN3A | NR3A | Inhibition | 17157509 |
| 3 | Teniposide intracellular | TOP2B | TOP2 beta | Inhibition | 8967966 |
| 4 | Etoposide intracellular | TOP2B | TOP2 beta | Inhibition | 1312600, 1312601, 1662724, 2158562, 2167985, 2537424, 2550587, 2849640, 7473578, 7922123, 8120864, 8295216, 8410993, 9211397, 10395485, 10809021, 11754608, 12877556, 15008514, 15084135, 15158802, 15177438, 16242334, 16903072, 17035025, 17580961, 14504921 |
| 5 | INO 1001 intracellular | PARP1 | PARP-1 | Inhibition | 15523000, 18535785, 20364863, 14523042 |
| 6 | Diazoxide intracellular | ABCC8 | SUR1 | Activation | 10419549, 11073882, 11121575, 12023875, 12565699, 14741296, 15561900 |
| 7 | Tedisamil extracellular region | ABCC8 | SUR1 | Inhibition | 10445672, 10684468, 10829253 |
| 8 | Glimepiride intracellular | ABCC8 | SUR1 | Inhibition | 9779817, 10773014, 11078468, 12819907, 20055691, 11325810 |
| 9 | Epirubicin intracellular | TOP2B | TOP2 beta | Inhibition | 16322310 |
| 10 | Annamycin intracellular | TOP2B | TOP2 beta | Inhibition | 15542779 |
| 11 | As(,2)O(,3) intracellular | PARP1 | PARP-1 | Inhibition | 12883267 |
| 12 | (R/S)-Repaglinide extracellular region | ABCC8 | SUR1 | Inhibition | 10773014, 11440368, 11716850, 12196472, 12623163, 12819907, 15200348, 15219283, 15380228, 15678092 |
| 13 | TOP53 intracellular | TOP2B | TOP2 beta | Inhibition | 11170388 |
| 14 | Acetohexamide extracellular region | ABCC8 | SUR1 | Activation | 15200348, 15561903 |
| 15 | Elsamitrucin intracellular | TOP2B | TOP2 beta | Inhibition | 8280493 |
| 16 | Ketamine extracellular region | GRIN3A | NR3A | Inhibition | 17084865, 8336337, 8941398, 9719604, 11937336 |
| 17 | NK109 intracellular | TOP2B | TOP2 beta | Inhibition | 9303354 |
| 18 | Tifenazoxide extracellular region | ABCC8 | SUR1 | Activation | 12213059, 12961066, 14514634, 14764798, 15220194 |
| 19 | Olaparib intracellular | PARP1 | PARP-1 | Inhibition | 18800822, 22343925, 23049934 |
| 20 | Intoplicine intracellular | TOP2B | TOP2 beta | Inhibition | 8043587 |

In one aspect, one or more compounds or a derivative thereof may be used to facilitate transfer of the nanoparticle. These include one or more of the following: (that can be administered to patients approximately 30 to 60 minutes prior to dosing with DNPs) doxorubicin, sildenafil androstanolone, acetohexamide, and teniposide, roscovitine (Imidazopyrimidine), spermine (Dialkylamine), geldanamycin (Macrolactam), ruxolitinib (Pyrrolopyrimidine), teniposide (Podophyllotoxin),sildenafil (Benzenesulfonamide), androstanolone (Anabolic Steroiod), acetohexamide (Alkyl-Phenylketone), doxorubicin (Anthracycline), sonolisib (Steroid Lactone), LY294002 (Benzopyran), TG100115 (Pteridine), Trifluoperazine (Benzothiazine, Phenothiazine), CEP5214 (Indole Derivative, Pyrrolocarbazole), Afuresertib (Substituted Benzene, Phenethylamine, Amphetamine), Cation Vemurafenib (Aryl-Phenylketone), Suramin (Benzene Derivative, Benzanilide), Uprosertib (Benzene Derivative, Phenethylamine, Amphetamine), Flutamide, (Benzene Derivative, Trifluoromethylbenzene), Enzastaurin (Indole Derivative, N-alkylindole), Fasudil (Isoquinoline derivative), Ruboxistaurin (Macrolactam), Pentamidine (Phenol Ether), Uprosertib (Benzene Derivative, Phenethylamine, Amphetamine), Lestaurtinib (Indole derivative, Indolocarbazole), Adenine (Imidazolepyrimidine), Pimozide (Diphenylbutylpiperidine), Chlorpromazine (Phenothiazine), and Afuresertib (Benzene Derivative, Phenethylamine, Amphetamine).

These individual compounds alone or in combination may be combined with the nanoparticle formulation or administered prior or post intranasal (IN) administration (e.g., 5 µg (with respect to DNA) in a 25 µl solution).

The gene transfer may occur in the context of administration to a cell in a human, i.e., administration of a vector containing a nucleic acid to a mammal, particularly a human. For example, an individual may be administered a compound and/or RNAi as disclosed herein prior to administration of a nucleic acid delivery system as known in the art (exemplary nucleic acid delivery systems are known in the art and disclosed in References 11-16). The nucleic acid may be single stranded or double stranded, or may, in certain instances, utilize multiple delivery vehicles which may employ one or the other or both.

The nanoparticle delivery vehicle may take a variety of forms. For example, in one aspect, the nucleic acid delivery vehicle may be a nanoparticle comprising said gene. In one aspect, the nucleic acid delivery vehicle may be a nanoparticle comprising a lysine polymer conjugated to PEG and complexed with a nucleic acid comprising the gene.

In one aspect, the proteins that inhibit the nucleic acid delivery vehicle uptake may be selected from keratin 13, APC protein, protocadherin 17, spectrin alph (non-erythrocytic 1), or a combination thereof.

In one aspect, a period of time exists between step a and step b. In aspects in which the nucleic acid delivery vehicle is administered following delivery of an RNAi and/or compound as disclosed herein, the nucleic acid delivery vehicle may be administered to an individual in need thereof, for example, 30 minutes, or 60 minutes, or 90 minutes, or 120 minutes following the administration of a compound and/or RNAi as disclosed herein. In the case of RNAi, in some aspects, the RNAi may be administered about 12 hours in advance of a nucleic acid delivery vehicle, about 20 hours in advance of a nucleic acid delivery vehicle, about 24 hours in advance of a nucleic acid delivery vehicle, or about 30 hours in advance of administration of the delivery vehicle.

For example, for RNAi application, patient stem cells or patient derived iPSCs are harvested and cultured and treated with RNAi against a gene in **Table 1** for 24 hr. NNPs formulated to contain an expression cassette for the therapeutic gene are then added to the cells for 72 hr. Reagents and delivery vector are replaced daily. An example of the time involved for the active agent application method is; patients are treated with one or more of the compounds claimed **Tables 2** and **3** about 30 to about 60 minutes prior to gene delivery vector administration. Agent treatment may be conducted one or more times before gene therapy. NNPs containing an expression cassette for the therapeutic gene may then be administered to the airways of the patient, for example, via nebulization.

In one aspect, the method may include the step of providing a reagent that facilitates transfection. In one aspect, said agent may be a cationic lipid transfection reagent (e.g. Lipofectamine or GL67), which may be mixed with a nucleic acid under a given formulation to produce a nucleic acid/lipid complex. For lipid (or protein) nucleic acid complexes, any formulation that produces lipid/nucleic acid or protein/nucleic acid complexes (of which there are 1000s) can be combined with the methods herein. This may similarly apply to protein polymers such PEGylated poly L lysine or PEI. For viral vectors, the vector may be produced in cell lines, purified and used for therapy in accordance with the disclosed methods.

Compositions comprising RNAi and/or the compounds of Tables 2 and/or 3 may be administered intranasally. In such aspect, the compositions may further comprise other agents suited for improved delivery across nasal mucosa. For example, in certain aspects, agents such as a permeation enhancer, a polymer capable of increasing mucosal adhesion of the composition, or a combination thereof may be included in the composition. In one aspect, the disclosed compositions may comprise, consist of or consist essentially of any of the aforementioned features, in any combination.

It will be appreciated by those skilled in the art that the particular method of administration will depend on a variety of factors, all of which are considered routinely when administering therapeutics, particularly in the context of gene transfer. It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, i.e., the mode of treatment and the daily number of doses o given for a defined number of days, can be ascertained by those skilled in the art using conventional treatment tests.

### Nanoparticle Counterions

Disclosed are nanoparticles containing nucleic acids such as DNA or RNA, which may be double or single stranded, and which may be protein coding or anti-sense coding or non-coding. The nucleic acids may include analogs of RNA and/or DNA (including, for example, miRNA, shRNA, tRNA, siRNA, single and double stranded DNA) that are modified to enhance degradation in vivo.

Methods of making nanoparticles in accordance with the instant invention are known in the art. See, for example US 8,017,577, entitled "Lyophilizable and enhanced compacted nucleic acids," and/or "Chapter 33: Real-Time Imaging of Gene Delivery and Expression with DNA Nanoparticle Technologies" by Sun and Ziady, filed herewith, both of which are incorporated herein in their entirety by reference. Disclosed herein are alternate counterions to those disclosed in the art which are used to manufacture nucleic acid nanoparticles. Counterions of polycations used to compact nucleic acids are known to affect the shape of particles formed. Shape is associated with nuclease resistance and colloidal stability. Moreover, shape affects the suitability and efficacy of compacted nucleic acid complexes for transfecting cells by various routes into a mammalian body.

The counterion that may be used in making compacted nucleic acid complexes may also have an effect on the stability of the complexes to lyophilization. Disclosed herein are nanoparticles which are compacted using one or more counterions selected from from trifluoroacetate (TFA), bromide, bicarbonate, glutamate, aspartate, hydroxyl ions, or combinations thereof, which may be used before compaction of the nucleic acid.

Polycations may comprise polyamino acids such as polylysine and derivatives of polylysine. The polycation may contain from 15-60 lysine residues, preferably in the ranges of 15-30, 30-45, or 45-60 residues. Exemplary derivatives of polylysine are CK15, CK30, CK45, which have an additional cysteine residue attached to polylysine polymers of length 15, 30, and 45 residues, respectively. Other amino acids can be readily attached to polylysine. Other polycationic amino acid polymers can be used such as polyarginine, or copolymers of arginine and lysine. Polymers of non-protein amino acids, such as omithine or citrulline, could also be used. Any pharmaceutically approved or appropriate polycation can be used including but not limited to protamine, histones, polycationic lipids, putrescine, spermidine, spermine, peptides, and polypeptides. The polycation may also contain a targeting moiety, which is typically a ligand which binds to a receptor on a particular type of cell. The targeting ligand may be a polyamino acid or other chemical moiety. Specificity of interaction of the ligand and the receptor is important for purposes of targeting. In one aspect, the polycation may be reacted with a bifunctional PEG (e.g. PEG-maleimide (PEG-Mal) or ortho-pyridyl disulfide (OPSS) (PEG-OPSS) to allow for the addition of a targeting moiety.

In one aspect, a composition is disclosed, the composition comprising a) a compacted nucleic acid nanoparticle as described above; and b) one or more agents selected from sonolisib (Steroid Lactone), LY294002 (Benzopyran), TG100115 (Pteridine), Trifluoperazine (Benzothiazine, Phenothiazine), CEP5214 (Indole Derivative , Pyrrolocarbazole), Afuresertib (Substituted Benzene, Phenethylamine, Amphetamine), Cation Vemurafenib (Aryl-Phenylketone), Suramin (Benzene Derivative, Benzanilide), Uprosertib (Benzene Derivative, Phenethylamine, Amphetamine), Flutamide, (Benzene Derivative, Trifluoromethylbenzene), Enzastaurin (Indole Derivative, N-alkylindole), Fasudil (Isoquinoline derivative), Ruboxistaurin (Macrolactam), Pentamidine (Phenol Ether), Uprosertib (Benzene Derivative, Phenethylamine, Amphetamine), Lestaurtinib (Indole derivative, Indolocarbazole), Adenine (Imidazolepyrimidine), Pimozide (Diphenylbutylpiperidine), Chlorpromazine (Phenothiazine), Afuresertib (Benzene Derivative, Phenethylamine, Amphetamine), and combinations thereof; and optionally, one or more agents selected from Table 1 or Table 2.

Kits are also provided. In one aspect, a kit may comprise or consist essentially of agents or compositions described herein. The kit may be a package that houses a container which may contain one or more compounds or solutions containing an RNAi as disclosed herein, and also houses instructions for administering the agent or composition to a subject. In one aspect, a pharmaceutical pack or kit is provided comprising one or more containers filled with one or more composition as disclosed herein. Associated with such container(s) can be various written materials such as instructions for use, or a notice in the form prescribed by a governmental agency regulating the manufacture, use or sale of pharmaceuticals or biological products, which notice reflects approval by the agency of manufacture, use, or sale for human administration.

As there may be advantages to mixing a component of a composition described herein and a pharmaceutically acceptable carrier, excipient or vehicle near the time of use, kits in which components of the compositions are packaged separately are disclosed. For example, the kit can contain an active ingredient in a powdered or other dry form in, for example, a sterile vial or ampule and, in a separate container within the kit, a carrier, excipient, or vehicle, or a component of a carrier, excipient, or vehicle (in liquid or dry form). In one aspect, the kit can contain a component in a dry form, typically as a powder, often in a lyophilized form in, for example, a sterile vial or ampule and, in a separate container within the kit, a carrier, excipient, or vehicle, or a component of a carrier, excipient, or vehicle. Alternatively, the kit may contain a component in the form of a concentrated solution that is diluted prior to administration. Any of the components described herein, any of the carriers, excipients or vehicles described herein, and any combination of components and carriers, excipients or vehicles can be included in a kit.

Optionally, a kit may also contain instructions for preparation or use (e.g., written instructions printed on the outer container or on a leaflet placed therein) and one or more devices to aid the preparation of the solution and/or its administration to a patient (e.g., one or a plurality of syringes, needles, filters, tape, tubing (e.g., tubing to facilitate intravenous administration) alcohol swabs and/or the Band-Aid^{®} applicator). Compositions which are more concentrated than those administered to a subject can be prepared. Accordingly, such compositions can be included in the kits with, optionally, suitable materials (e.g., water, saline, or other physiologically acceptable solutions) for dilution. Instructions included with the kit can include, where appropriate, instructions for dilution.

In other embodiments, the kits can include pre-mixed compositions and instructions for solubilizing any precipitate that may have formed during shipping or storage. Kits containing solutions of one or more of the aforementioned active agents, or pharmaceutically acceptable salts thereof, and one or more carriers, excipients or vehicles may also contain any of the materials mentioned above (e.g., any device to aid in preparing the composition for administration or in the administration per se). The instructions in these kits may describe suitable indications (e.g., a description of patients amenable to treatment) and instructions for administering the solution to a patient.

### Examples

### Method for enhancing nucleic acid transfer

Applicant has discovered methods for enhancing the efficiency of gene transfer through the use of interference RNA (RNAi) technology or pharmacological agents that modulate the interactome (FIG 1) of nucleic acid nanoparticles consisting of polymers of lysine conjugated to PEG and complexed with nucleic acids. Both of these approaches have been reduced to practice and achieve significantly higher levels of gene transfer in the context of condensed DNA nanoparticle vectors, resulting in as much as 50-fold greater gene transfer efficiency. These technologies represent a significant enhancement to gene transfer technologies.

By using a novel immunocapture procedure (FIG 2), Applicant identified protein interactors of polyethylene glycol conjugated DNA nanoparticles. This investigation revealed 474 unique proteins that interact with the nanoparticles as listed in Table 3. Many of these proteins represent a nanoparticle specific transfection interactome, but a number of proteins such as Prohibitin 1 and 2 are also involved in viral as well as liposomal gene delivery. Some of these protein interactors may be inhibiting the cellular uptake of DNA nanoparticles as well as other vectors for the delivery of nucleic acids. The interactome segregated into sites in the cell where nucleic acid particles are delivered (Table 4). In this method, Applicant used RNAi and/or pharmacological agents to modulate the particle interactome and enhance nucleic acid delivery to the nucleus (DNA) or the ribosome (RNA).

**Table 4. Characteristics of the nucleic acid nanoparticle cellular protein interactome**

| **Rank** | **Cellular Processes** | **Class** | **Cellular Localization** | **P Value** | **False Discovery Rate** | **Percent of Dataset (%)** |
|---|---|---|---|---|---|---|
| 1 | Intermediate filaments | Cytoskeleton | Cytosol | 1.99E-14 | 8.68E-13 | 21.75 |
| 2 | Translation initiation | Translation | Ribosome | 1.05E-16 | 1.37E-14 | 15.45 |
| 3 | Elongation-Termination | Translation | Ribosome | 1.99E-15 | 1.30E-13 | 12.47 |
| 4 | Actin filaments | Cytoskeleton | Cytosol | 4.84E-08 | 1.58E-06 | 10.01 |
| 5 | Chromatin modification | Transcription | Nucleus | 7.80E-06 | 2.04E-04 | 9.71 |
| 6 | Spindle microtubules | Cytoskeleton | Cytosol | 7.16E-04 | 8.53E-03 | 8.08 |
| 7 | mRNA processing | Transcription | Nucleus | 1.06E-04 | 1.83E-03 | 7.71 |
| 8 | Cell junctions | Cell adhesion | Cell membrane | 1.21E-04 | 1.83E-03 | 7.61 |
| 9 | Regulation of cytoskeleton rearrangement | Cytoskeleton | Cytosol | 1.25E-04 | 1.83E-03 | 7.22 |

For RNAi application, RNAi molecules may be delivered to the cells, or in the case of delivery to an individual, to the individual, prior to the desired nucleic acid delivery vehicle. The RNAi molecules are administered in an amount sufficient to target and knock down specific cellular proteins that negatively impact the uptake of the nucleic acid delivery vehicle. RNAi decreases the cellular levels of these proteins, reducing their deleterious impact on the downstream transfer of nucleic acids. RNAi mediated knockdown of four of these proteins has been tested by Applicant, which resulted in significant enhancement of gene transfer in ¾ constructs tested. RNAi targeted to interfere with the synthesis of the 4 proteins; keratin 13 (GI: 81891678), APC protein (GI: 97535708), protocadherin 17 (GI:94538350), and spectrin alpha (non-erythrocytic 1, GI:119608216) that are deleterious to gene transfer with the DNA nanoparticles improved gene delivery (FIG 3).

In addition to, or separately, pharmacological agents that modulate the DNP interactome can enhance nucleic acid transfer to the nucleus or the ribosome (in the case of RNA delivery). Applicant found 13 compounds and their derivatives that modulate 71 interactors (see Table 2) that can be administered to patients about 30 to about 60 minutes prior to dosing with DNPs. These are classified by cellular site of action. For example, Doxorubicin and Sildenafil will act to inhibit or promote interactions in the cytosol. Androstanolone will modulate interactions at the ribosome. Acetohexamide will promote non-nucleolin mediated interactions at the cell membrane. Ruxolitinib and Teniposide may be used to modulate nuclear interactions. Applicant's data also points to the importance to the interaction with nucleolin and how modulation of this interaction at the plasma membrane greatly impacts gene transfer with DNPs (FIGS 3-5). Modulation of these cellular interactions is expected to have different impacts on RNPs vs. DNPs, as the cellular compartment targets for these formulations of NNPs vary (ribosome vs. nucleus, respectively). For example, drugs that promote cellular entry may benefit both DNPs and RNPs. However, drugs that diminish interactions at the ribosome would be expected to only benefit DNPs. Conversely, drugs that diminish nuclear interactions should benefit RNPs.

**Table 2 and 3** outlines compounds may be used to modulate nucleolin associated nucleic acid nanoparticle (NNP) uptake. Nucleolin translocation to the cell surface may be promoted by IP injections of roscovitine (inhibits CDK1 at 10 mg/kg), spermine (induces CKII at 50 mg/kg), geldanamycin (inhibits HSP90 interaction with nucleolin at 15 mg/kg), or hydrocortisone (increases GR shuttling of nucleolin to the cell surface at 7.5 mg/kg) into animals 60 min prior to a 25 µl intranasal (IN) administration of 5 µg (with respect to DNA) NNPs containing the CFTR gene, as has been previously reported(1). Control groups injected with either DMSO instead of pharmacological agents, and NNPs containing the transgene with no drug may be used. CF mice may receive NPD measurements 1 week before treatment (a background/baseline measurement) and then 4, 7, and 14 days after transfection with CFTR-containing NNPs applied to the nose, as previously reported (1). Two weeks following transfection mice will be sacrificed, and the lungs may be harvested, paraffin imbeded, and sectioned for immunohistochemistry, and sections probed with the CF3 or 24:1 anti-CFTR Ab that does not cross react with mouse cftr, as previously reported (1). Studies can be duplicated in F508del and S489X homozygote mice.

Use in Research. The RNAi and/or pharmacological approaches to enhancing gene transfer may be developed as an additive to current gene transfer and transfection vectors. For example, it may be used as a supplemental additive to the cationic lipid transfection reagent Lipofectamine, enabling either greater gene transfer or decreased amounts of transfection reagent used, resulting in either reduced costs or enhanced efficiency. Alternatively, pharmacological and RNAi treatment may be employed prior to or concurrent with the delivery of viral vectors in in vitro or ex vivo gene transfer applications. This may allow more cellular gene modification and higher expression of therapeutic transgenes within these cells, or decreased viral titer needed to provide curative levels of cell modification. This may increase the efficacy of these genes or reduce the associated costs with producing sufficient amount of virus, which is currently a significant obstacle in gene therapy protocols.

Use in human therapy. CF is the most common inherited recessive disorders in Caucasians, and advances in small molecule therapy have not significantly benefitted a large majority of the patients. Gene therapy (repair or replacement) offers a potential of corrective therapy for the disease regardless of mutation type. The disclosed methods may be useful for enhancing corrective DNA and/or RNA delivery with a synthetic vector to airway epithelial cells, the most affected cells in CF. The present example relates to the biology of NNPs, a vector that has been shown to have partial efficacy in correcting CFTR in CF patients (2). Applicant has found 71 specific protein interactors (for example, some of the interactors and associated regulation are shown in FIG 2, others are listed in **Table 1)** that help define the biology of the particles in cells and can be targeted with 13 FDA approved drugs **(Table 2).** Other compounds are listed in **Table 3.**

Applicant has demonstrated that modulating the NNP interactome can enhance gene transfer by 10-50 fold, the highest levels of enhancement ever achieved in two decades of modifying and examining DNP-based vectors (see FIGS 3 and 4). Based on this result and given the fact that DNPs have achieved partial clinical correction in a Phase 1 trial in CF patients (2), the methods of the instant disclosure have the potential to provide pharmacological agents that can enhance gene transfer to fully therapeutic levels in humans. While airway epithelial cells are the primary site of disease and the most important gene therapy target in CF, a better understanding of the determinants of successful gene transfer into these cells will significantly benefit gene delivery for a number of other diseases, including chronic obstructive pulmonary disease (COPD; ~12,000,000 patients in the USA), and epithelial lung cancers (~200,000 patients in the USA). The instant disclosure provides a novel approach to implementation of NNP biology. Findings in airway epithelia will likely be relevant to other cell targets where NNPs have succeeded, including cells in the brain (3-6) and retina (7-10), and may be relevant to the cellular uptake of other non-viral polyplex-based vectors as well as viral and liposomal vectors.

In a therapeutic context, siRNA can be applied to human cells *ex vivo* or pharmacological agents to humans directly before or during gene delivery to optimize gene transfer obtained with DNA/RNA nanoparticles, and potential with liposomal and viral vectors as well.

### Reference List

1. Ziady AG, Kelley TJ, Milliken E, Ferkol T, Davis PB. Functional evidence of CFTR gene transfer in nasal epithelium of cystic fibrosis mice in vivo following luminal application of DNA complexes targeted to the serpin-enzyme complex receptor. Mol.Ther. 2002 Apr;5(4):413-9
2. Konstan MW, Davis PB, Wagener JS, Hilliard KA, Stern RC, Milgram LJ, Kowalczyk TH, Hyatt SL, Fink TL, Gedeon CR, et al. Compacted DNA nanoparticles administered to the nasal mucosa of cystic fibrosis subjects are safe and demonstrate partial to complete cystic fibrosis transmembrane regulator reconstitution. Hum.Gene Ther. 2004 Dec;15(12):1255-69
3. Yurek DM, Fletcher AM, Smith GM, Seroogy KB, Ziady AG, Molter J, Kowalczyk TH, Padegimas L, Cooper MJ. Long-term transgene expression in the central nervous system using DNA nanoparticles. Mol.Ther. 2009 Apr;17(4):641-50
4. Yurek DM, Flectcher AM, Kowalczyk TH, Padegimas L, Cooper MJ. Compacted DNA nanoparticle gene transfer of GDNF to the rat striatum enhances the survival of grafted fetal dopamine neurons. Cell Transplant. 2009;18(10):1183-96. PMCID:PMC3031110
5. Yurek DM, Fletcher AM, McShane M, Kowalczyk TH, Padegimas L, Weatherspoon MR, Kaytor MD, Cooper MJ, Ziady AG. DNA Nanoparticles: Detection of Long-term Transgene Activity In Brain Using Bioluminescence Imaging. Mol.Imaging 2011 Apr 26;
6. Fletcher AM, Kowalczyk TH, Padegimas L, Cooper MJ, Yurek DM. Transgene expression in the striatum following intracerebral injections of DNA nanoparticles encoding for human glial cell line-derived neurotrophic factor. Neuroscience 2011 Oct 27;194:220-6. PMCID:PMC3408714
7. Farjo R, Skaggs J, Quiambao AB, Cooper MJ, Naash MI. Efficient non-viral ocular gene transfer with compacted DNA nanoparticles. PLoS.One. 2006;1:e38. PMCID:PMC1762345
8. Ding XQ, Quiambao AB, Fitzgerald JB, Cooper MJ, Conley SM, Naash MI. Ocular delivery of compacted DNA-nanoparticles does not elicit toxicity in the mouse retina. PLoS.One. 2009;4(10):e7410. PMCID:PMC2756629
9. Cai X, Conley SM, Nash Z, Fliesler SJ, Cooper MJ, Naash MI. Gene delivery to mitotic and postmitotic photoreceptors via compacted DNA nanoparticles results in improved phenotype in a mouse model of retinitis pigmentosa. FASEB J. 2010 Apr;24(4):1178-91. PMCID:PMC2845431
10. Koirala A, Makkia RS, Conley SM, Cooper MJ, Naash MI. S/MAR-containing DNA nanoparticles promote persistent RPE gene expression and improvement in RPE65-associated LCA. Hum.Mol.Genet. 2013 Apr 15;22(8):1632-42. PMCID:PMC3605833
11. Ziady AG, Davis PB, Konstan MW. Non-viral gene transfer therapy for cystic fibrosis. Expert.Opin.Biol.Ther. 2003 Jun;3(3):449-58
12. Ziady AG, Davis PB. Current prospects for gene therapy of cystic fibrosis. Curr.Opin.Pharmacol. 2006 Oct;6(5):515-21
13. Ahmed H, Shubina-Oleinik O, Holt JR. Emerging Gene Therapies for Genetic Hearing Loss. J.Assoc.Res.Otolaryngol. 2017 Aug 16;
14. Naso MF, Tomkowicz B, Perry WL, III, Strohl WR. Adeno-Associated Virus (AAV) as a Vector for Gene Therapy. BioDrugs. 2017 Jul 1. PMCID:PMC5548848
15. Huang J, Wang Y, Zhao J. CRISPR Editing in Biological and Biomedical Investigation. J.Cell Physiol 2017 Aug 8;
16. Zhang X, Wang L, Liu M, Li D. CRISPR/Cas9 system: a powerful technology for in vivo and ex vivo gene therapy. Sci.China Life Sci. 2017 May;60(5):468-75

All percentages and ratios are calculated by weight unless otherwise indicated.

All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "20 mm" is intended to mean "about 20 mm."

Every document cited herein, including any cross referenced or related patent or application, is hereby incorporated herein by reference in its entirety unless expressly excluded or otherwise limited. All accessioned information (e.g., as identified by PUBMED, PUBCHEM, NCBI, UNIPROT, or EBI accession numbers) and publications in their entireties are incorporated into this disclosure by reference in order to more fully describe the state of the art as known to those skilled therein as of the date of this disclosure. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications may be made without departing from the spirit and scope of the invention. It is therefore intended to cover in the appended claims all such changes and modifications that are within the scope of this invention.
Aspects of the invention will now be described with reference to the following numbered clauses:
1. A method for transferring a gene into a eukaryotic cell, comprising administering a compacted nucleic acid nanoparticle; and one or more agents selected from sonolisib (Steroid Lactone), LY294002 (Benzopyran), TG100115 (Pteridine), Trifluoperazine (Benzothiazine, Phenothiazine), CEP5214 (Indole Derivative , Pyrrolocarbazole), Afuresertib (Substituted Benzene, Phenethylamine, Amphetamine), Cation Vemurafenib (Aryl-Phenylketone), Suramin (Benzene Derivative, Benzanilide), Uprosertib (Benzene Derivative, Phenethylamine, Amphetamine), Flutamide, (Benzene Derivative, Trifluoromethylbenzene), Enzastaurin (Indole Derivative, N-alkylindole), Fasudil (Isoquinoline derivative), Ruboxistaurin (Macrolactam), Pentamidine (Phenol Ether), Uprosertib (Benzene Derivative, Phenethylamine, Amphetamine), Lestaurtinib (Indole derivative, Indolocarbazole), Adenine (Imidazolepyrimidine), Pimozide (Diphenylbutylpiperidine), Chlorpromazine (Phenothiazine), Afuresertib (Benzene Derivative, Phenethylamine, Amphetamine), and combinations thereof, to a eukaryotic cell.
2. The method of clause 1, further comprising administering an inhibitor of a protein that inhibits nanoparticle delivery uptake, said inhibitor being selected from one or more of RNAi, miRNA, shRNA, tRNA, siRNA, single stranded DNA, double stranded DNA, and combinations thereof, and wherein said nucleic acid inhibits synthesis of one or more proteins that inhibit nucleic acid delivery vehicle uptake, preferably wherein said one or more proteins is selected from Table 1.
3. The method of clause 1 or clause 2, further comprising administering an active agent that facilitates compacted nucleic acid nanoparticle uptake into a cell, wherein said active agent inhibits synthesis of one or more proteins that inhibit nucleic acid delivery vehicle uptake.
4. The method of clause 1, wherein said RNAi molecule inhibits expression of a gene encoding a protein selected from Table 1.
5. The method of any preceding clause, further comprising administering a second active agent selected from an agent listed in Table 2 or Table 3.
6. The method of any preceding clause, wherein said active agent is selected from roscovitine, geldanamycin, acetohexamide, and ruxolitinib, or a combination thereof.
7. The method of any preceding clause, wherein said nucleic acid delivery vehicle is a nanoparticle comprising said gene.
8. The method of any preceding clause, wherein said compacted nucleic acid nanoparticle comprises a nucleic acid plasmid and a polymer, wherein said nanoparticle is compacted in the presence of a counter ion selected from trifluoroacetate (TFA), bromide, bicarbonate, glutamate, hydroxyl ions or combinations thereof.
9. The method of clause 8, wherein said nucleic acid is single or double stranded DNA.
10. The method of clause 8 or 9, wherein said polymer is a polycation.
11. The method of any of clauses 8 through 10, wherein said polycation is a lipid.
12. The method of any of clauses 8 through 11, wherein said polycation is a cysteine (C) containing polymer of lysine (K), such as CK30, a cysteine (C) containing polymer of arginine (R), such as CR30, or combinations thereof.
13. The method of any of clauses 8 through 12, wherein said polycation is selected from a cysteine (C) containing polymer of lysine (K) and arginine (R), , or polymers of lysine mixed with arginine, conjugated to PEG and complexed with nucleic acids
14. The method of any of clauses 8 through 13, wherein said polymer is a lysine polymer, preferably a polyethylene glycol (PEG)-substituted lysine polymer or polyethylenemine.
15. The method of any preceding clause, wherein said compacted nucleic acid nanoparticle has a shape selected from rod shape, ellipsoidal, spheroidal, or toroidal.
16. The method of any preceding clause, wherein said compacted nucleic acid nanoparticle particles have a diameter of 25-400 nm in length as measured by electron microscopy.
17. A composition comprising
   a) a compacted nucleic acid nanoparticle as described in any preceding clause 8 through 16; and
   b) one or more agents selected from sonolisib (Steroid Lactone), LY294002 (Benzopyran), TG100115 (Pteridine), Trifluoperazine (Benzothiazine, Phenothiazine), CEP5214 (Indole Derivative , Pyrrolocarbazole), Afuresertib (Substituted Benzene, Phenethylamine, Amphetamine), Cation Vemurafenib (Aryl-Phenylketone), Suramin (Benzene Derivative, Benzanilide), Uprosertib (Benzene Derivative, Phenethylamine, Amphetamine), Flutamide, (Benzene Derivative, Trifluoromethylbenzene), Enzastaurin (Indole Derivative, N-alkylindole), Fasudil (Isoquinoline derivative), Ruboxistaurin (Macrolactam), Pentamidine (Phenol Ether), Uprosertib (Benzene Derivative, Phenethylamine, Amphetamine), Lestaurtinib (Indole derivative, Indolocarbazole), Adenine (Imidazolepyrimidine), Pimozide (Diphenylbutylpiperidine), Chlorpromazine (Phenothiazine), Afuresertib (Benzene Derivative, Phenethylamine, Amphetamine), and combinations thereof; and optionally, one or more agents selected from Table 1 or Table 2.

## Claims

1. A nucleic acid delivery vehicle containing a nucleic acid that encodes the cystic fibrosis (CF) gene or an active portion of the CF gene for use in a method of treating cystic fibrosis, wherein the nucleic acid delivery vehicle is administered concurrently with, before, or after administration of i) a compound selected from the group consisting of roscovitine, ruxolitinib, spermine, cortisone, resveratrol, doxorubicin, acetohexamide, sildenafil, geldanamycin, alvespimycin, entasobulin, androstanolone, quercetin, and teniposide and/or ii) an RNAi that inhibits expression of a gene encoding a protein selected from Table 1.

2. The nucleic acid delivery vehicle for use of claim 1, wherein the compound is selected from the group consisting of roscovitine, ruxolitinib, spermine, cortisone, resveratrol, doxorubicin, acetohexamide, and sildenafil.

3. The nucleic acid delivery vehicle for use of claim 1 or 2, wherein the compound is selected from the group consisting of roscovitine, ruxolitinib, and spermine.

4. The nucleic acid delivery vehicle for use of any one of claims 1-3, wherein the compound is roscovitine or ruxolitinib.

5. The nucleic acid delivery vehicle for use of any one of claims 1-4, wherein the RNAi inhibits expression of a gene encoding a protein selected from the group consisting of APC protein, keratin 13, protocadherin 17, and alpha II-spectrin.

6. The nucleic acid delivery vehicle for use of any one of claims 1-5, wherein the nucleic acid delivery vehicle is a DNA nanoparticle (DNP).

7. The nucleic acid delivery vehicle for use of any one of claims 1-6, wherein the nucleic acid delivery vehicle is a DNP comprising a lysine polymer conjugated to polyethylene glycol (PEG) and complexed with the nucleic acid.

8. The nucleic acid delivery vehicle for use of any one of claims 1-7, wherein the compound is administered about 30 to about 60 minutes prior to administration of the nucleic acid delivery vehicle.

9. The nucleic acid delivery vehicle for use of any one of claims 1-8, wherein the RNAi is administered about 12 hours, about 20 hours, about 24 hours, or about 30 hours prior to administration of the nucleic acid delivery vehicle.

10. The nucleic acid delivery vehicle for use of any one of claims 1-9, wherein the compound is administered at a dose of from about 0.001 mM to about 100 mM.

11. The nucleic acid delivery vehicle for use of any one of claims 1-10, wherein the RNAi and/or the compound are administered intranasally.
